# FASCICULE DE BREVET EUROPEEN

(11) **EP 0 607 355 B1**
(45) Date de publication et mention de la délivrance du brevet: **22.11.1995**
(21) Numéro de dépôt: 92922727.0
(22) Date de dépôt: 08.10.1992
(51) Int. Cl.: C07D 243/24, C07D 405/12, C07D 403/12, C07D 401/04, C07F 9/645, A61K 31/55, A61K 31/675

(54) **3-UREIDO-BENZODIAZEPINONES UTILES COMME ANTAGONISTES DE CCK OU DE GASTRINE**
3-UREIDO-BENZODIAZEPINONE VERWENDBAR ALS CCK- ODER GASTRIN-ANTAGONISTEN
3-UREIDO-BENZODIAZEPINONES USEFUL AS ANTAGONISTS OF CCK OR OF GASTRINE

(30) Priorité: 10.10.1991 FR 9112481
(43) Date de publication de la demande: 27.07.1994
(73) Titulaire: RHONE-POULENC RORER S.A., 92160 Antony (FR)
(72) Inventeur: CAPET, Marc, F-94320 Thiais (FR); COTREL, Claude, F-75012 Paris (FR); DUBRUCQ, Marie-Christine, F-95880 Enghien-les-Bains (FR); GUYON, Claude, F-94100 Saint-Maur-des-Fosses (FR); MARTIN, Jean-Paul, F-92700 Colombes (FR)
(74) Mandataire: Savina, Jacques
(86) Numéro de dépôt international: FR9200935
(87) Numéro de publication internationale: WO9307130

(56) Documents cités:
- EP-A- 0 434 360
- EP-A- 0 434 364
- EP-A- 0 434 369
- EP-A- 0 508 796
- EP-A- 0 508 797

## Description

La présente invention concerne des dérivés de formule : 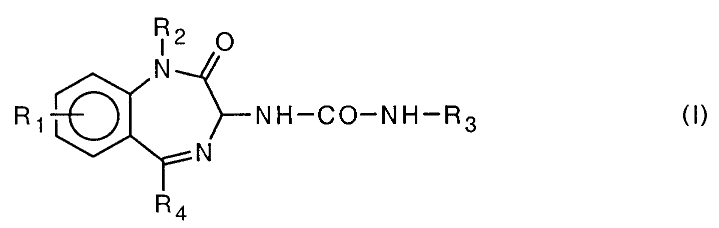
leurs sels, leur préparation et les médicaments les contenant.

Des dérivés de benzodiazépine, antagonistes de la CCK sont décrits dans le brevet EP 434369. D'autres benzodiazépines antagonistes de la CCK sont également décrites dans les demandes de brevet intercalaires (Article 54(3) CBE) EP 508796 et EP 508797.

Dans la formule (I),
- R₁ représente un atome d'hydrogène ou d'halogène ou un radical alkyle, alcoxy, alkylthio, nitro, hydroxy ou cyano,
- R₂ représente un radical alkyle ou une chaîne -CH(R₅)-CO-R₆ dans laquelle R₅ représente un atome d'hydrogène ou un radical alkyle, alcoxycarbonyle ou phényle éventuellement substitué (par un ou plusieurs substituants choisis parmi les atomes d'halogène et les radicaux alkyle, alcoxy, alkylthio et nitro) et R₆ représente un radical alcoxy, cycloalkyloxy éventuellement substitué (par au moins un radical alkyle), cycloalkylalkyloxy, phénylalkyloxy, polyfluoroalkyloxy, cinnamyloxy, un reste -NR₇R₈ dans lequel R₇ et R₈ identiques ou différents représentent un atome d'hydrogène ou un radical alkyle, phényle éventuellement substitué (par un ou plusieurs substituants choisis parmi les atomes d'halogène et les radicaux alkyle, alcoxy et alkylthio), cycloalkylalkyle, cycloalkyle, indanyle, phénylalkyle ou bien R₇ et R₈ forment ensemble avec l'atome d'azote auquel ils sont rattachés un hétérocycle mono ou polycyclique saturé ou insaturé contenant 4 à 9 atomes de carbone et un ou plusieurs hétéroatomes (O, S, N) et éventuellement substitué par un ou plusieurs radicaux alkyle, alcoxy, alcoxycarbonyle, dialkylcarbamoyle, phényle ou par un cycle spiromonocyclique formé par combinaison d'un atome de l'hétérocycle avec 4 ou 5 autres atomes de carbone dont l'un ou plusieurs de ces derniers peuvent éventuellement être remplacés par des hétéroatomes (O, S, N),
- R₃ représente (a) un radical phényle substitué par un ou plusieurs substituants choisis parmi les radicaux -alk-SO₃H, -alk-PO₃H₂, -CH=NOH, -CH=NO-alk-COOX, -S-alk-COOX, -SO-alk-COOX, -SO₂-alk-COOX, -CH=CH-COOX, -alk-CO-NHOH, -C(=NOH)-COOX, -alk-N(OH)-CO-alk, -alk-SO₂H, -CH=CH-SO₃H, -C(COOX)=N-O-alk-COOX, tétrazolylalkyle ou un groupe de formule : 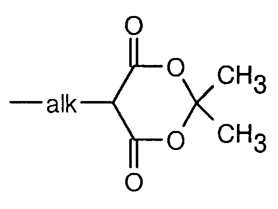 ou (b) un cycle de formule : 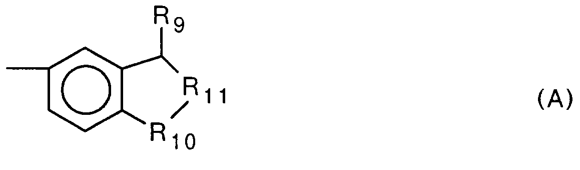 dans laquelle R₉ représente un radical =NOX, =NO-alk-COOX, =CH-COOX, -alk-COOX, -alk-SO₂H ou -alk-SO₃H, R₁₀ représente un atome d'oxygène ou de soufre ou un radical méthylène ou alkylimino et R₁₁ représente un radical méthylène ou éthylène,
- R₄ représente un radical pyridyle ou phényle éventuellement substitué par un ou plusieurs substituants choisis parmi les atomes d'halogène et les radicaux alkyle, alcoxy, hydroxy, carboxy et nitro,
- alk représente un radical alkyle ou alkylène,
- X représente un atome d'hydrogène ou un radical alkyle,
à l'exception des composés pour lesquels R₁ représente un atome d'hydrogène ou 1 ou 2 halogène ou méthyle, R₂ représente un radical alkyle ou -CH(R₅)-CO-R₆ dans lequel R₅ représente un atome d'hydrogène et R₆ représente un reste pyrrolidinyl-1, R₃ représente un radical phényle substitué en position 3 par un radical tétrazolyl-5 méthyle et R₄ représente un radical phényle éventuellement substitué par un ou deux atomes d'halogène ou méthyle.

Dans les définitions qui précèdent et celles qui seront citées ci-après, sauf mention contraire, les radicaux alkyle, alkylène et alcoxy et les portions alkyle, alkylène et alcoxy contiennent 1 à 4 atomes de carbone en chaîne droite ou ramifiée, et les radicaux et portions cycloalkyle contiennent 3 à 12 atomes de carbone.

Lorsque R₇ et R₈ forment avec l'atome d'azote auquel ils sont rattachés un hétérocycle, celui-ci est de préférence un cycle pipéridino, perhydroazépinyl-1, tétrahydro-1,2,3,6 pyridyl-1, tétrahydro-1,2,3,4 quinolyl-1, pyrrolidinyl-1, tétrahydro-1,2,3,4 isoquinolyl-2, morpholino, thiomorpholino ou indolyl-1, ces cycles pouvant être éventuellement substitués par un ou plusieurs radicaux alkyle.

Les composés de formule (I) comportant un ou plusieurs centres asymétriques présentent des formes isomères. Les racémiques et les énantiomères de ces composés font également partie de l'invention.

Les composés de formule (I) peuvent être préparés par action d'un dérivé réactif de l'acide carbamique, obtenu éventuellement in situ par action d'un dérivé réactif de l'acide carbonique choisi parmi le N,N'-diimidazole carbonyle, le phosgène, le chloroformiate de trichlorométhyle, le carbonate de bis(trichlorométhyle) ou le chloroformiate de p-nitrophényle sur un dérivé de formule : 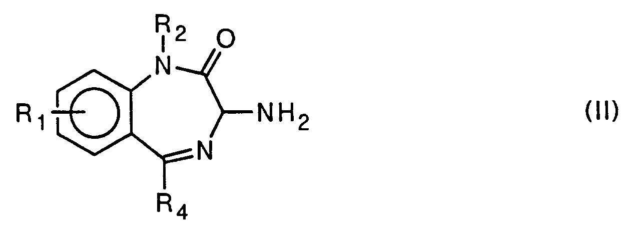
dans laquelle R₁, R₂ et R₄ ont les mêmes significations que dans la formule (I), sur un dérivé de formule :

H₂N-R₃ (III)

dans laquelle R₃ a les mêmes significations que dans la formule (I) ou, lorsque R₃ comporte un reste COOH, PO₃H₂, SO₂H ou SO₃H, un sel d'un tel composé.

Cette réaction s'effectue de préférence au sein d'un solvant inerte tel que le tétrahydrofuranne, le diméthylformamide, un solvant chloré (chloroforme, dichloro-1,2 éthane par exemple), un solvant aromatique (benzène, toluène par exemple), à une température comprise entre 20°C et la température d'ébullition du solvant. Comme sels, on utilise de préférence un sel avec un métal alcalin (sodium, potassium par exemple) ou un sel de tétraalkylammonium (tétrabutylammonium par exemple).

Les dérivés de formule (II) peuvent être obtenus par application ou adaptation des méthodes décrites dans les brevets US 3371084, US 3652634, les demandes de brevet DE 3907390, NL 327674, EP 349949 et EP 385735, par I. FRYER, bicyclic diazepines, diazepine with an additionnal ring, John Wiley & sons Inc., M. G. BOCK et coll., J. Org. Chem., 52, 3232 (1987) et dans les exemples.

Les dérivés de formule (III) sont commercialisés ou peuvent être obtenus par application ou adaptation des méthodes décrites par R. SCHRÖTER, Methoden des organischen Chemie, Houben Weil, band XI/1, 360 et dans les exemples.

Les composés de formule (I) peuvent être également préparés par action d'un dérivé de formule : 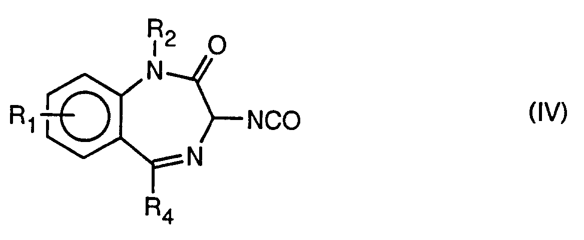
dans laquelle R₁, R₂ et R₄ ont les mêmes significations que dans la formule (I), sur une amine de formule (III) ou, lorsque R₃ comporte un reste COOH, PO₃H₂, SO₂H ou SO₃H, un sel d'un tel composé.

Cette réaction s'effectue généralement au sein d'un solvant inerte tel que le tétrahydrofuranne, le diméthylformamide, un solvant chloré (chloroforme, dichloro-1,2 éthane par exemple), un solvant aromatique (benzène, toluène par exemple), à une température comprise entre 10°C et la température d'ébullition du solvant.

Les dérivés de formule (IV) peuvent être obtenus par action d'un dérivé de formule (II) sur un dérivé réactif de l'acide carbonique tel que le phosgène, le chloroformiate de trichlorométhyle ou le carbonate de bis(trichlorométhyle).

Cette réaction s'effectue au sein d'un solvant inerte tel que le toluène, à une température comprise entre -30°C et 110°C

Les composés de formule (I) à l'exception de ceux pour lesquels R₃ représente soit un radical phényle substitué par -alk-PO₃H₂, -CH=NOH, -CH=NO-alk-COOX, -S-alk-COOX, -SO-alk-COOX, -SO₂-alk-COOX, -CH=CH-COOX, -alk-CO-NHOH, -alk-N(OH)-CO-alk, -C(=NOH)-COOX, -C(COOX)=NO-alk-COOX, -alk-SO₂H ou tétrazolylalkyle, soit un cycle de formule (A) dans laquelle R₉ représente un radical =NOX, =NO-alk-COOX, =CH-COOX, -alk-COOX ou -alk-SO₂H et X représente un atome d'hydrogène peuvent également être préparés par action d'un dérivé de formule (II), sur un isocyanate de formule :

OCN-R₃ (V)

dans laquelle R₃ a les mêmes significations que précédemment.

Cette réaction s'effectue généralement au sein d'un solvant inerte tel que le tétrahydrofuranne, le diméthylformamide, un solvant chloré (chloroforme, dichloro-1,2 éthane par exemple), un solvant aromatique (benzène, toluène par exemple), à une température comprise entre 10°C et la température d'ébullition du solvant.

Les isocyanates de formule (V) peuvent être obtenus par application ou adaptation de la méthode décrite par R. RICHTER et col., The Chemistry of Cyanate and their thio derivatives, S. PATAI, part 2, Wiley New-York (1977) et des méthodes décrites dans les exemples.

Les composés de formule (I) pour lesquels R₃ représente un radical phényle substitué par un radical -SO-alk-COOX ou SO₂-alk-COOX et X représente un radical alkyle peuvent être préparés par oxydation des composés de formule (I) correspondants pour lesquels R₃ représente un radical phényle substitué par un radical -S-alk-COOX.

Cette oxydation s'effectue généralement au moyen d'oxone^{R} (peroxymonosulfate de potassium) commercialisé par Aldrich, au sein d'un alcool tel que le méthanol ou un mélange méthanol-eau, à une température voisine de 25°C.

Les composés de formule (I) pour lesquels R₃ représente soit un radical phényle substitué par -CH=N-O-alk-COOX, -S-alk-COOX, -SO-alk-COOX, -C(=NOH)-COOX, -SO₂-alk-COOX, -CH=CH-COOX ou -C(COOH)=N-O-alk-COOX, soit un cycle de formule (A) dans laquelle R₉ représente un radical =CH-COOX, =NO-alk-COOX ou -alk-COOX et X représente un atome d'hydrogène peuvent également être préparés par coupure d'un ester correspondant.

Cette coupure s'effectue par toutes méthodes connues de l'homme de l'art pour transformer un ester en acide. Lorsque l'on utilise un ester d'alkyle, il est avantageux d'opérer soit au moyen d'une base telle que la lithine, la soude ou la potasse, au sein d'un solvant inerte tel que le tétrahydrofuranne, le dioxanne, la pyridine, l'eau, un alcool ou un mélange de ces solvants, à une température comprise entre 20°C et la température d'ébullition du solvant, soit au moyen d'acide trifluoroacétique éventuellement au sein d'un solvant inerte tel qu'un solvant chloré (dichlorométhane, chloroforme, dichloro-1,2 éthane par exemple), à une température comprise entre 20°C et la température d'ébullition du solvant. Lorsque l'on utilise un ester benzylique, il est avantageux d'opérer au moyen d'hydrogène, en présence d'un métal de transition, au sein d'un solvant inerte tel qu'un alcool, à une température comprise entre 20 et 40°C ou selon la méthode décrite par T. TSUJI et coll., Tetrahedron Letters, 2793 (1979).

Les composés de formule (I) pour lesquels R₃ représente un cycle (A) dans lequel R₉ représente un radical =NOX ou =NO-alk-COOX peuvent également être préparés par action d'un dérivé de formule : 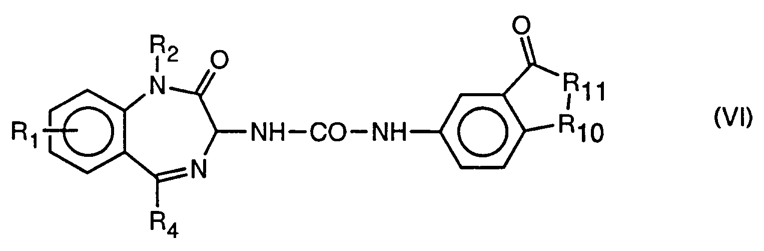
dans laquelle R₁, R₂, R₄, R₁₀ et R₁₁ ont les mêmes significations que dans la formule (I), sur un dérivé de formule :

H₂NOZ (VII)

dans laquelle Z représente un atome d'hydrogène, un radical alkyle ou -alk-COOX.

Cette réaction s'effectue de préférence au sein d'un solvant tel que la pyridine, l'eau ou un mélange de ces solvants, à la température d'ébullition du milieu réactionnel.

Il est entendu pour l'homme de métier que, pour la mise en oeuvre des procédés selon l'invention décrits précédemment, il peut être nécessaire d'introduire des groupes protecteurs des fonctions amino, hydroxy, carboxy afin d'éviter des réactions secondaires tels que ceux décrits par T. W. GREENE, Protective Groups in Organic Synthesis, John Wiley, New York. Les fonctions amino peuvent par exemple être bloquées sous forme de carbamates de tert-butyle ou de méthyle puis régénérées au moyen d'iodotriméthylsilane ou de carbamates de benzyle puis régénérées par hydrogénation après avoir mis en oeuvre le procédé selon l'invention. Les fonctions hydroxy peuvent par exemple être bloquées sous forme de benzoate puis régénérées par hydrolyse en milieu alcalin après avoir mis en oeuvre le procédé selon l'invention.

Les énantiomères des composés de formule (I) contenant au moins un site asymétrique peuvent être obtenus par dédoublement des racémiques par exemple par chromatographie sur colonne chirale selon W.H. PIRKLE et coll., asymetric synthesis, vol. 1, Academic Press (1983) ou sur colonne de silice enrobée de tris diméthyl-3,5 phénylcarbamate de cellulose telles que celles décrites dans J. Am. Chem. Soc, 106, 5357 (1984) en éluant avec un solvant convenable tel que l'éthanol ou un mélange éthanol-hexane ou par synthèse à partir des précurseurs chiraux.

Les composés de formule (I) peuvent être purifiés par les méthodes connues habituelles, par exemple par cristallisation, chromatographie ou extractions.

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste acide peuvent également être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'un tétraalkylammonium, d'une amine ou d'un sel d'un acide organique sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) présentent des propriétés pharmacologiques intéressantes. Ces composés possèdent une forte affinité pour les récepteurs de la cholécystokinine (CCK) et de la gastrine et sont donc utiles dans le traitement et la prévention des désordres liés à la CCK et à la gastrine au niveau du système nerveux et de l'appareil gastrointestinal.

C'est ainsi que ces composés peuvent être utilisés pour le traitement ou la prévention des psychoses, des troubles anxieux, des attaques de panique, de la maladie de Parkinson, de la diskynésie tardive, du syndrôme du colon irritable, de la pancréatite aiguë, des ulcères, des désordres de la motilité intestinale, de certaines tumeurs sensibles à la CCK, comme régulateur de l'appétit, dans le sevrage aux traitements chroniques et abus d'alcool ou de médicaments et pour controler la constriction de la pupille de l'oeil.

Ces composés ont également un effet de potentialisation sur l'activité analgésique des médicaments narcotiques et non narcotiques. En outre, ils peuvent avoir un effet analgésique propre.

Par ailleurs, les composés ayant une forte affinité pour les récepteurs CCK modifient les capacités de mémorisation. En conséquence, ces composés peuvent être efficaces dans les troubles de la mémoire.

L'affinité des composés de formule (I) pour les récepteurs CCK a été déterminée selon une technique inspirée de celle de A. SAITO et coll . (J. Neuro. Chem., 37, 483-490 (1981)) au niveau du cortex cérébral et au niveau du pancréas.

Dans ces tests, la CI₅₀ des composés de formule (I) est généralement inférieure ou égale à 1000 nM.

Par ailleurs, il est connu que les produits qui reconnaissent les récepteurs centraux de la CCK ont une spécificité similaire pour les récepteurs de la gastrine dans le tractus gastrointestinal (BOCK et coll., J. Med. Chem., 32, 16-23 (1989); REYFELD et coll., Am. J. Physiol., 240, G255-266 (1981); BEINFELD et coll., Neuropetides, 3, 411-427 (1983)).

Les composés de formule (I) présentent une toxicité faible. Leur DL₅₀ est généralement supérieure à 40 mg/kg par voie sous cutanée chez la souris.

D'un intérêt particulier, sont les composés de formule (I) pour lesquels R₁ représente un atome d'hydrogène, R₂ représente un radical alkyle ou -CH(R₅)COR₆ dans lequel R₅ représente un atome d'hydrogène et R₆ représente un radical -NR₇R₈ dans lequel R₇ et R₈ forment ensemble avec l'atome d'azote auquel ils sont rattachés un hétérocycle et en particulier un cycle pyrrolidinyl-1 ou pipéridino éventuellement substitués par un ou plusieurs radicaux alkyle, R₃ représente (a) un radical phényle substitué par un ou plusieurs substituants choisis parmi les radicaux -alk-SO₃H, -alk-PO₃H₂, -CH=NOH, -CH=NO-alk-COOX, -S-alk-COOX, -SO-alk-COOX, -SO₂-alk-COOX, -CH=CH-COOX, -alk-CO-NHOH, -C(=NOH)-COOX, -alk-N(OH)-CO-alk, -alk-SO₂H, -CH=CH-SO₃H, -C(COOX)=N-O-alk-COOX, tétrazolylalkyle, ou un groupe de formule : 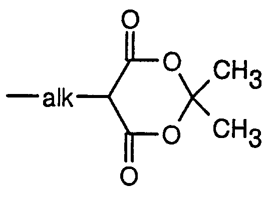
ou (b) un cycle de formule : 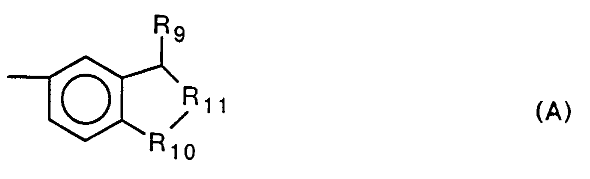
dans laquelle R₉ représente un radical =NOX, =NO-alk-COOX, =CH-COOX, -alk-COOX, -alk-SO₂H ou -alk-SO₃H, R₁₀ représente un atome d'oxygène ou de soufre ou un radical méthylène ou alkylimino et R₁₁ représente un radical méthylène ou éthylène et R₄ représente un radical phényle.

Sont particulièrement intéressants les composés suivants :
- acide {{[dihydro-2,3 oxo-2 phényl-5 (pyrrolidinyl-1)carbonylméthyl-1 1H-benzo[f]diazépine-1,4 yl-3]-3 uréido}-3 phénylthio}acétique-(RS),
- acide [(dihydro-2,3 méthyl-1 oxo-2 phényl-5 1H-benzo[f]diazépine-1,4 yl-3)-3 uréido]-3 phénylméthanesulfonique-(RS),
- acide {[(N-méthyl N-phényl-carbamoylméthyl)-1 oxo-2 phényl-5 dihydro-2,3 1H-benzo[f]diazépine-1,4 yl-3]-3 uréido}-3 phényl-3 propène-2(E)oïque-(RS),
- acide {{[dihydro-2,3 oxo-2 phényl-5 (diméthyl-3,3 pipéridino)carbonylméthyl-1 1H-benzo[f] diazépine-1,4 yl-3]-3 uréido}-3 phénylthio}acétique-(RS),
- acide {{[(N,N-diéthyl-carbamoylméthyl)-1 dihydro-2,3 oxo-2 phényl-5 1H-benzo[f]diazépine-1,4 yl-3]-3 uréido}-3 phénylthio}acétique-(RS),
- N-(dihydro-2,3 méthyl-1 oxo-2 phényl-5 1H-benzo[f]diazépine-1,4 yl-3) N'-(hydroxyimino-1 indanyl-6) urée-(E)-(RS),
- acide {dihydro-3,4 [(dihydro-2,3 méthyl-1 oxo-2 phényl-5 1H-benzo[f]diazépine-1,4 yl-3)-3 uréido]-6 2H-benzopyrannylidène-4}-2 acétique-(E)-(RS),
- acide {[dihydro-2,3 oxo-2 phényl-5 (pyrrolidinyl-1)carbonylméthyl-1 1H-benzo[f]diazépine-1,4 yl-3]-3 uréido}-3 phénylméthylènaminooxy-2 acétique-(E)-(RS),
- acide {{[(dihydro-2,3 oxo-2 phényl-5 (pyrrolidinyl-1)carbonylméthyl-1 1H-benzo[f]diazépine-1,4 yl-3]-3 uréido}-3 phényl}-2 (carboxyméthyloxyimino)-2 acétique-(RS),
- {{[dihydro-2,3 oxo-2 phényl-5 (pyrrolidinyl-1)carbonylméthyl-1 1H-benzo[f]diazépine-1,4 yl-3]-3 uréido}-3 benzyl}-5 diméthyl-2,2 dioxanne-1,3 dione-4,6-(RS),
- acide {{[(N,N-diéthyl-carbamoylméthyl)-1 dihydro-2,3 oxo-2 phényl-5 1H-benzo[f]diazépine-1,4 yl-3]-3 uréido}-3 phényl}-2 éthylènesulfonique-(E)-(RS),
- {{[(N,N-diéthyl-carbamoylméthyl)-1 dihydro-2,3 oxo-2 phényl-5 1H-benzo[f]diazépine-1,4 yl-3]-3 uréido}-3 benzyl}-5 tétrazole-(RS),
- acide {[(N,N-diéthyl-carbamoylméthyl)-1 dihydro-2,3 oxo-2 phényl-5 1H-benzo[f]diazépine-1,4 yl-3]-3 uréido}-3 alpha-hydroxyimino phénylacétique-(E)-(RS),
- acide {{[(N,N-diéthyl-carbamoylméthyl)-1 dihydro-2,3 (fluoro-2 phényl)-5 oxo-2 1H-benzo[f]diazépine-1,4 yl-3]-3 uréido}-3 phénylthio}acétique-(RS),
- acide {{[dihydro-2,3 (diméthyl-3,3 pipéridino)carbonylméthyl-1 oxo-2 phényl-5 1H-benzo[f]diazépine-1,4 yl-3]-3 uréido}-4 phénylthio}acétique-(RS),
- acide {[dihydro-2,3 oxo-2 phényl-5 (diméthyl-3,3 pipéridino)carbonylméthyl-1 1H-benzo[f]diazépine-1,4-yl-3]-3 uréido}-3 phényl}-3 propène-2 oïque-(E)-(RS),
- acide {{[dihydro-2,3 oxo-2 phényl-5 (pyrrolidinyl-1)carbonylméthyl-1 1H-benzo[f]diazépine-1,4 yl-3]-3 uréido}-4 phénylthio}acétique-(RS),
- {[(dihydro-2,3 oxo-2 phényl-5 méthyl-1 1H-benzo[f]diazépine-1,4 yl-3-(RS))-3 uréido]-3 phényl}-1 éthanesulfonate de potassium-(RS),
- acide (+)-{{[dihydro-2,3 oxo-2 phényl-5 (pyrrolidinyl-1)carbonylméthyl-1 1H-benzo[f]diazépine-1,4 yl-3]-3 uréido}-3 phénylthio}acétique,
- acide (-)-{{[dihydro-2,3 oxo-2 phényl-5 (pyrrolidinyl-1)carbonylméthyl-1 1H-benzo[f]diazépine-1,4 yl-3]-3 uréido}-3 phénylthio}acétique,
- acide {{[dihydro-2,3 oxo-2 phényl-5 (pyrrolidinyl-1)carbonylméthyl-1 1H-benzo[f]diazépine-1,4 yl-3-(RS)]-3 uréido}-3 phénylsulfinyl}acétique-(RS),
- acide {{[dihydro-2,3 oxo-2 phényl-5 (pyrrolidinyl-1)carbonylméthyl-1 1H-benzo[f]diazépine-1,4 yl-3]-3 uréido}-3 phénylsulfonyl}acétique-(RS),
- acide {{[dihydro-2,3 oxo-2 phényl-5 (pyrrolidinyl-1)carbonylméthyl-1 1H-benzo[f]diazépine-1,4 yl-3-(RS)]-3 uréido}-3 phènylthio}-2 propionique-(RS).

Les exemples suivants illustrent l'invention sans la limiter.

### EXEMPLE 1

Une solution de 1 g de {{[dihydro-2,3 oxo-2 phényl-5 (pyrrolidinyl-1)carbonylméthyl-1 1H-benzo[f]diazépine-1,4 yl-3]-3 uréido}-3 phénylthio}acétate de tert-butyle-(RS) dans 4 cm3 d'acide trifluoroacétique est agitée à une température voisine de 20°C pendant 20 heures puis versée dans 200 cm3 d'eau glacée. Le solide est séparé par filtration et rincé par 20 cm3 d'eau puis 20 cm3 d'oxyde de diisopropyle. On obtient, après recristallisation dans l'éthanol, 0,65 g d'acide {{[dihydro-2,3 oxo-2 phényl-5 (pyrrolidinyl-1)carbonylméthyl-1 1H-benzo[f]diazépine-1,4 yl-3]-3 uréido}-3 phénylthio}acétique-(RS) fondant à 168°C.

Le {{[dihydro-2,3 oxo-2 phényl-5 (pyrrolidinyl-1)carbonylméthyl-1 1H-benzo[f]diazépine-1,4 yl-3]-3 uréido}-3 phénylthio}acétate de tert-butyle-(RS) peut être obtenu de la manière suivante : à une solution de 3,2 g d'amino-3 dihydro-2,3 oxo-2 phényl-5 (pyrrolidinyl-1 carbonylméthyl)-1 1H-benzo[f]diazépine-1,4-(RS) dans 40 cm3 de tétrahydrofuranne, on ajoute, à une température voisine de 20°C, une solution de 2,6 g d'(isocyanato-3 phénylthio)acétate de tert-butyle dans 10 cm3 de tétrahydrofuranne. Le mélange est agité pendant 70 heures à une température voisine de 20°C puis concentré à sec sous pression réduite (2,7 kPa) à 40°C. Le résidu est dissous dans 30 cm3 de diméthylformamide. La solution est versée dans 200 cm3 d'eau glacée et le solide est filtré, séché et chromatographié sur 200 cm3 de silice (éluant : acétate d'éthyle). Les fractions contenant le produit attendu sont réunies et concentrées à sec sous pression réduite (2,7 kPa). On obtient ainsi 3,9 g de {{[dihydro-2,3 oxo-2 phényl-5 (pyrrolidinyl-1)carbonylméthyl-1 1H-benzo[f]diazépine-1,4 yl-3]-3 uréido}-3 phénylthio}acétate de tert-butyle-(RS) sous forme d'un solide amorphe utilisé tel quel dans les synthèses ultérieures.

L'amino-3 dihydro-2,3 oxo-2 phényl-5 (pyrrolidinyl-1)carbonylméthyl-1 1H-benzo[f]diazépine-1,4-(RS) peut être obtenue de la manière suivante : une solution de 5,1 g de dihydro-2,3 hydroxyimino-3 oxo-2 phényl-5 (pyrrolidinyl-1)carbonylméthyl-1 1H-benzo[f]diazépine-1,4 dans 50 cm3 de méthanol est ajoutée à une suspension de 1 g de ruthénium à 5 % sur charbon dans 35 cm3 de méthanol. Le mélange est agité pendant 30 heures à une température voisine de 70°C sous atmosphère d'hydrogène (500 kPa). Le catalyseur est séparé par filtration et le filtrat concentré sous pression réduite (2,7 kPa). Le résidu est dissous dans 100 cm3 de dichlométhane et extrait par 3 fois 50 cm3 d'une solution aqueuse normale d'acide chlorhydrique. Les phases aqueuses sont réunies et amenées à un pH voisin de 9 par addition d'hydrogénocarbonate de sodium puis extraites par 3 fois 100 cm3 de dichlorométhane. Les phases organiques sont réunies, séchées sur sulfate de magnésium, filtrées et concentrées sous pression réduite (2,7 kPa). On obtient ainsi 3,25 g d'amino-3 dihydro-2,3 oxo-2 phényl-5 (pyrrolidinyl-1)carbonylméthyl-1 1H-benzo[f]diazépine-1,4-(RS) sous forme d'une meringue utilisée telle quelle dans les synthèses ultérieures.

La dihydro-2,3 hydroxyimino-3 oxo-2 phényl-5 (pyrrolidinyl-1) carbonylméthyl-1 1H-benzo[f]diazépine-1,4 peut être préparée de la manière suivante : à une solution de 7,27 g de tert-butylate de potassium dans 50 cm3 de tert-butanol et 150 cm3 de tétrahydrofuranne, on ajoute, à une température voisine de -20°C, 9 g de dihydro-2,3 oxo-2 phényl-5 (pyrrolidinyl-1)carbonylméthyl-1 1H-benzo[f]diazépine-1,4, par portions, en 50 minutes. La suspension est agitée pendant 3 heures à une température inférieure à -20°C puis additionnée de 9,8 cm3 de nitrite d'isopentyle. La suspension est encore agitée pendant 1 heure à une température voisine de 0°C puis on ajoute 5 cm3 d'acide acétique et 10 cm3 d'eau. Après retour à une température voisine de 20°C, le précipité est filtré et lavé au pentane. On obtient ainsi, après recristallisation dans l'éthanol, 3,17 g de dihydro-2,3 hydroxyimino-3 oxo-2 phényl-5 (pyrrolidinyl-1)carbonylméthyl-1 1H-benzo[f]diazépine-1,4 fondant au dessus de 260°C.

La dihydro-2,3 oxo-2 phényl-5 (pyrrolidinyl-1)carbonylméthyl-1 1H-benzo[f]diazépine-1,4 peut être obtenue de la manière suivante : une suspension de 25 g de dihydro-2,3 oxo-2 phényl-5 1H-benzo[f]diazépine-1,4, de 20,5 g de chloroacétyl-1 pyrrolidine, de 14,6 g de carbonate de potassium et de 17,6 g d'iodure de potassium dans 250 cm3 de diméthylformamide est agitée pendant 170 heures à une température voisine de 25°C. Le mélange réactionnel est versé dans 1500 cm3 d'eau glacée. La phase aqueuse est séparée de l'insoluble et extraite par 3 fois 300 cm3 d'acétate d'éthyle. Les extraits organiques sont réunis, lavés par 200 cm3 d'une solution aqueuse saturée en chlorure de sodium, séchés sur sulfate de magnésium, filtrés et concentrés à sec sous pression réduite (2,7 kPa) à une température voisine de 45°C. On obtient ainsi après cristallisation dans l'acétate d'éthyle 14,7 g de dihydro-2,3 oxo-2 phényl-5 (pyrrolidinyl-1)carbonylméthyl-1 1H-benzo[f]diazépine-1,4 fondant à 190°C.

L'(isocyanato-3 phénylthio)acétate de tert-butyle peut être obtenu de la manière suivante : à une suspension de 0,24 g de charbon dans 1,46 cm3 de chloroformiate de trichlorométhyle et 15 cm3 de toluène, on ajoute goutte à goutte, à une température voisine de -30°C, une solution de 2,9 g d'(amino-3 phénylthio)acétate de tert-butyle dans 25 cm3 de toluène. Le milieu réactionnel est agité pendant 2 heures à une température voisine de 20°C puis pendant 2 heures et 30 minutes à une température voisine de 110°C. Le mélange réactionnel est ensuite refroidi à une température voisine de 20°C, dégazé par barbottage d'azote, filtré puis concentré à sec sous pression réduite (2,7 kPa) à 50°C. On obtient ainsi 2,6 g d'(isocyanato-3 phénylthio)acétate de tert-butyle sous forme d'une huile utilisée telle quelle dans les synthèses ultérieures.

L'(amino-3 phénylthio)acétate de tert-butyle peut être préparé de la manière suivante : une solution de 25 g d'amino-3 thiophénol et de 39 g de bromoacétate de tert-butyle dans 400 cm3 d'éthanol est agitée pendant 3 heures à une température voisine de 25°C puis concentrée sous pression réduite (2,7 kPa). Le résidu est dissous dans 500 cm3 d'acétate d'éthyle. La solution est lavée successivement par 3 fois 75 cm3 d'une solution aqueuse normale d'hydroxyde de sodium, 3 fois 75 cm3 d'une solution aqueuse saturée de chlorure de sodium et concentrée sous pression réduite (2,7 kPa). Le résidu est ensuite purifié par chromatographie sur 800 cm3 de silice (éluant : cyclohexane - acétate d'éthyle (90-10 puis 80-20 en volumes)). Les fractions contenant le produit attendu sont réunies et concentrées sous pression réduite (2,7 kPa). On obtient ainsi 24 g d'(amino-3 phénylthio)acétate de tert-butyle sous forme d'une huile utilisée telle quelle dans les synthèses ultérieures.

La chloroacétyl-1 pyrrolidine peut être préparée selon la méthode décrite par A. J. SPEZIALE et coll., J. Am. Chem. Soc., 78, 2556 (1956).

La dihydro-2,3 oxo-2 phényl-5 1H-benzo[f]diazépine-1,4 peut être préparée par la méthode décrite par L.H. STERNIBACH et coll., J. Org. Chem., 27, 3788 (1962).

### EXEMPLE 2

A une solution de 1,8 g de N,N'-diimidazole carbonyle dans 30 cm3 de dichloro-1,2 éthane, on ajoute à une température voisine de 25°C, une solution de 2,65 g d'amino-3 dihydro-2,3 méthyl-1 oxo-2 phényl-5 1H-benzo[f]diazépine-1,4-(RS) dans 50 cm3 de dichloro-1,2 éthane. Le mélange réactionnel est agité pendant 3 heures à une température voisine de 25°C puis on ajoute une solution de 4,3 g d'amino-3 phénylméthane sulfonate de tétrabutylammonium dans 70 cm3 de dichloro-1,2 éthane. Le mélange réactionnel est agité à une température voisine de 80°C pendant 18 heures puis refroidi à une température voisine de 25°C et dilué par 150 cm3 de dichlorométhane, lavé par 2 fois 100 cm3 d'eau, séché sur sulfate de magnésium, filtré et concentré à sec sous pression réduite (2,7 kPa) à 60°C. Une fraction de 2,8 g du résidu est purifiée par chromatographie sur 60 cm3 de silice contenus dans une colonne de 2 cm de diamètre (éluant : dichlorométhane - méthanol (97-3 en volumes)). Le résidu est dissous dans 50 cm3 de méthanol et traité par 10 g de résine acide (IR 120). La suspension est filtrée et lavée par 3 fois 50 cm3 de méthanol puis le filtrat est concentré sous pression réduite et le résidu trituré dans 50 cm3 d'oxyde de diéthyle, filtré et lavé par 2 fois 25 cm3 d'oxyde de diéthyle. On obtient ainsi 0,9 g d'acide [(dihydro-2,3 méthyl-1 oxo-2 phényl-5 1H-benzo[f]diazépine-1,4 yl-3)-3 uréido]-3 phénylméthanesulfonique-(RS) fondant à 230°C.

L'amino-3 phénylméthane sulfonate de tétrabutylammonium peut être préparé de la manière suivante : à une solution de 41,4 g de nitro-3 phénylméthanesulfonate de tétrabutylammonium dans 300 cm3 d'éthanol, on ajoute 1 g de palladium sur charbon à 5 %. La suspension est agitée pendant 2 heures à une température voisine de 20°C sous atmosphère d'hydrogène (128 kPa). Le catalyseur est séparé par filtration et le filtrat est concentré à sec sous pression réduite (2,7 kPa) à 50°C. On obtient ainsi 42,2 g d'amino-3 phénylméthanesulfonate de tétrabutylammonium sous forme d'une huile utilisée telle quelle dans les synthèses ultérieures.

Le nitro-3 phénylméthanesulfonate de tétrabutylammonium peut être préparé de la manière suivante : à 800 cm3 d'une solution aqueuse de dihydrogénophosphate de potassium 0,5 M, on ajoute 6,9 g de nitro-3 phénylméthanesufonate de sodium puis 9,9 g d'hydrogénosulfate de tétrabutyl ammonium. Le mélange est extrait par 500 cm3 de chlorure de méthylène. La phase organique est séchée sur sulfate de magnésium et concentrée à sec sous pression réduite à 40°C. On obtient ainsi 13 g de nitro-3 phénylméthanesulfonate de tétrabutylammonium sous forme d'une huile utilisée telle quelle dans les synthèses ultérieures.

Le nitro-3 phénylméthane sulfonate de sodium peut être préparé selon la technique décrite par PURGOTTI et col., Gazz. Chim. Ital., 30, II, 247.

L'amino-3 dihydro-2,3 méthyl-1 oxo-2 phényl-5 1H-benzo[f]diazépine-1,4-(RS) peut être obtenue selon la technique décrite par M. G. BOCK et coll., J. Org. Chem., 52, 3232 (1987).

### EXEMPLE 3

A une suspension de 1,7 g de {[(N-méthyl N-phényl-carbamoylméthyl)-1 oxo-2 phényl-5 dihydro-2,3 1H-benzo[f]diazépine-1,4 yl-3]-3 uréido}-3 phényl-3 propène-2 oate d'éthyle-(E)-(RS) dans 6 cm3 d'éthanol, on ajoute 3,4 cm3 d'une solution aqueuse normale d'hydroxyde de sodium et porte à une température voisine de 80°C pendant 30 minutes. Après refroidissement à une température voisine de 20°C, on ajoute 20 cm3 d'eau puis lave avec 50 cm3 d'acétate d'éthyle. La phase aqueuse est acidifiée à pH 1 avec 4 cm3 d'une solution aqueuse normale d'acide chlorhydrique. Après extraction par 50 cm3 de dichlorométhane, la phase organique est séchée sur sulfate de magnésium et concentrée sous pression réduite (2,7 kPa). On obtient après recristallisation dans l'oxyde de diisopropyle 0,6 g d'acide {[(N-méthyl N-phényl-carbamoylméthyl)-1 oxo-2 phényl-5 dihydro-2,3 1H-benzo[f]diazépine-1,4 yl-3]-3 uréido}-3 phényl-3 propène-2 oïque-(E)-(RS) fondant à 180°C.

Le {[(N-méthyl N-phényl-carbamoylmèthyl)-1 oxo-2 phényl-5 dihydro-2,3 1H-benzo[f]diazépine-1,4 yl-3]-3 uréido}-3 phényl-3 propène-2 oate d'éthyle-(E)-(RS) peut être préparé de la manière suivante : à une solution de 1,57 g d'(amino-3 dihydro-2,3 oxo-2 phényl-5 1H-benzo[f]diazépine-1,4 yl-1)-2 N-méthyl acétanilide-(RS) dans 20 cm3 de tétrahydrofuranne, on ajoute, sous atmosphère inerte, à une température voisine de 20°C, en 15 minutes, une solution de 0,85 g d'(isocyanato-3 phényl)-3 propène-2 oate-(E) d'éthyle dans 10 cm3 de tétrahydrofuranne. Le milieu réactionnel est agité à une température voisine de 20°C pendant 4 heures. Après concentration à sec sous pression réduite (2,7 kPa) à 50°C, le résidu est dissous dans 10 cm3 d'acétate d'éthyle puis on dilue avec 30 cm3 d'oxyde de diéthyle. On obtient ainsi 1,7 g de {[(N-méthyl N-phényl-carbamoylméthyl)-1 oxo-2 phényl-5 dihydro-2,3 1H-benzo[f]diazépine-1,4 yl-3]-3 uréido}-3 phényl-3 propène-2 oate d'éthyle-(E)-(RS) fondant à 238°C.

L'(isocyanato-3 phényl)-3 propène-2 oate-(E) d'éthyle peut être préparé de la manière suivante : sous atmosphère inerte, on refroidit à une température voisine de -20°C, une suspension de 0,15 g de charbon dans une solution de 1 g de carbonate de bis(trichlorométhyle) dans 10 cm3 de toluène. On coule en 15 minutes, en conservant la température à -20°C, une solution de 1,2 g de méta-amino cinnamate d'éthyle-(E) dans 10 cm3 de toluène. Le milieu réactionnel est agité 2 heures à une température voisine de 20°C puis 2 heures et 30 minutes à une température voisine de 110°C. On refroidit le milieu réactionnel à une température voisine de 20°C, filtre le noir sur clarcel, rince avec 20 cm3 de dichlorométhane et concentre sous pression réduite (2,7 kPa) pour obtenir 1,35 g d'(isocyanato-3 phényl)-3 propène-2 oate-(E) d'éthyle sous forme d'une huile utilisée telle quelle dans les synthèses ultérieures.

L'(amino-3 dihydro-2,3 oxo-2 phényl-5 1H-benzo[f]diazépine-1,4 yl-1)-2 N-méthyl-acétanilide-(RS) peut être préparé de la manière suivante : une suspension de 10 g de nickel de Raney dans une solution de 5,3 g de (dihydro-2,3 hydroxyimino-3 oxo-2 phényl-5 1H-benzo[f]diazépine-1,4 yl-1)-2 N-méthyl-acétanilide dans 75 cm3 de méthanol est agitée pendant 24 heures à une température voisine de 20°C, sous atmosphère d'hydrogène (1,5 MPa). Le catalyseur est séparé par filtration et le filtrat concentré sous pression réduite (2,7 kPa) à 35°C. Le résidu est purifié par chromatographie sur 400 cm3 de silice contenus dans une colonne de 4 cm de diamètre (éluants : dichlorométhane puis dichlorométhane-méthanol (98-2 puis 95-5 en volumes)). Les fractions contenant le produit attendu sont réunies et concentrées sous pression réduite (2,7 kPa). On obtient ainsi 1 g d'(amino-3 dihydro-2,3 oxo-2 phényl-5 1H-benzo[f]diazépine-1,4 yl-1)-2 N-méthylacétanilide-(RS) fondant à 80°C.

Le (dihydro-2,3 hydroxyimino-3 oxo-2 phényl-5 1H-benzo[f]diazépine-1,4 yl-1)-2 N-méthyl-acétanilide peut être préparé d'une manière analogue à celle décrite à l'exemple 1 pour la préparation de la dihydro-2,3 hydroxyimino-3 oxo-2 phényl-5 (pyrrolidinyl-1)carbonylméthyl-1 1H-benzo[f]diazépine-1,4, mais à partir de 5,04 g de tert-butylate de potassium, de 30 cm3 de tert-butanol, de 130 cm3 de tétrahydrofuranne, de 6,5 g de (dihydro-2,3 oxo-2 phényl-5 1H-benzo[f]diazépine-1,4 yl-1)-2 N-méthyl-acétanilide et de 3,2 cm3 de nitrite d'isopentyle. On obtient ainsi 5,3 g de (dihydro-2,3 hydroxyimino-3 oxo-2 phényl-5 1H-benzo[f]diazépine-1,4 yl-1)-2 N-méthyl-acétanilide sous forme d'une meringue utilisée telle quelle dans les synthèses ultérieures.

Le (dihydro-2,3 oxo-2 phényl-5 1H-benzo[f]diazépine-1,4 yl-1)-2 N-méthylacétanilide peut être obtenu d'une manière analogue à celle décrite à l'exemple 1 pour la préparation de la dihydro-2,3 oxo-2 phényl-5 (pyrrolidinyl-1)carbonylméthyl-1 1H-benzo[f]diazépine-1,4 , mais à partir de 4,6 g de N-méthyl bromo-2 acétanilide, de 4,7 g de dihydro-2,3 oxo-2 phényl-5 1H-benzo[f]diazépine-1,4, de 3,5 g de carbonate de potassium et de 50 cm3 de diméthylformamide. On obtient ainsi 8 g de (dihydro-2,3 oxo-2 phényl-5 1H-benzo[f]diazépine-1,4 yl-1)-2 N-méthyl-acétanilide utilisé tel quel dans les synthèses ultérieures.

Le N-méthyl bromo-2 acétanilide peut être préparé de la manière suivante : à une solution de 10,7 g de N-méthylaniline dans 65 cm3 de dichlorométhane, on ajoute successivement, à une température voisine de -5°C, 11,1 g de triéthylamine et une solution de 20,4 g de bromure de bromacétyle dans 10 cm3 de dichlorométhane. La suspension est agitée pendant 2 heures à une température voisine de 20°C puis additionnée de 25 cm3 d'eau. La phase aqueuse est séparée par décantation et réextraite par 2 fois 15 cm3 de dichlorométhane. Les phases organiques sont réunies, lavées par 3 fois 25 cm3 d'eau, séchées sur sulfate de magnésium, filtrées puis concentrées à sec sous pression réduite (2,7 kPa) à 40°C. L'huile résiduelle est additionnée de 100 cm3 d'éther diéthylique; le produit insoluble est séparé par filtration et lavé par 3 fois 15 cm3 d'éther diéthylique. Les filtrats sont réunis et concentrés à sec sous pression réduite (2,7 kPa) à 40°C. On obtient ainsi 20,5 g de N-méthyl bromo-2 acétanilide sous forme d'une huile utilisée telle quelle dans les synthèses ultérieures.

Le méta-amino cinnamate d'éthyle-(E) peut être obtenu par la méthode décrite dans la demande de brevet NL 7416449 (C.A., 84, 58882q).

### EXEMPLE 4

En opérant comme à l'exemple 1, mais à partir de 1,5 g de {{[dihydro-2,3 oxo-2 phényl-5 (diméthyl-3,3 pipéridino)carbonylméthyl-1 1H-benzo[f] diazépine-1,4 yl-3]-3 uréido}-3 phénylthio}acétate de tert-butyle-(RS) et de 5,5 cm3 d'acide trifluoroacétique, on obtient 0,55 g d'acide {{[dihydro-2,3 oxo-2 phényl-5 (diméthyl-3,3 pipéridino)carbonylméthyl-1 1H-benzo[f] diazépine-1,4 yl-3]-3 uréido}-3 phénylthio}acétique-(RS) fondant à 240°C.

Le {{[dihydro-2,3 oxo-2 phényl-5 (diméthyl-3,3 pipéridino)carbonylméthyl-1 1H-benzo[f]diazepine-1,4 yl-3]-3 uréido}-3 phénylthio}acétate de tert-butyle-(RS) peut être préparé d'une manière analogue à celle décrite à l'exemple 1 pour la préparation du {{[dihydro-2,3 oxo-2 phényl-5 (pyrrolidinyl-1)carbonylméthyl-1 1H-benzo[f]diazépine-1,4 yl-3}-3 uréido}-3 phénylthio)acétate de tert-butyle-(RS), mais à partir de 1,65 g d'amino-3 dihydro-2,3 oxo-2 phényl-5 (diméthyl-3,3 pipéridino)carbonylméthyl-1 1H-benzo[f]diazépine-1,4-(RS) et de 1,13 g d'(isocyanato-3 phénylthio)acétate de tert-butyle dans 35 cm3 de tétrahydrofuranne. On obtient ainsi 1,57 g de {{[dihydro-2,3 oxo-2 phényl-5 (diméthyl-3,3 pipéridino)carbonylméthyl-1 1H-benzo[f]diazépine-1,4 yl-3]-3 uréido}-3 phénylthio}acétate de tert-butyle-(RS) fondant à 110°C.

L'amino-3 dihydro-2,3 oxo-2 phényl-5 (diméthyl-3,3 pipéridino) carbonylméthyl-1 1H-benzo[f]diazépine-1,4-(RS) peut être préparée d'une manière analogue à celle décrite à l'exemple 1 pour la préparation de l'amino-3 dihydro-2,3 oxo-2 phényl-5 (pyrrolidinyl-1)carbonylméthyl-1 1H-benzo[f]diazépine-1,4-(RS), mais à partir de 7,1 g de dihydro-2,3 (diméthyl-3,3 pipéridino)carbonylméthyl-1 hydroxyimino-3 oxo-2 phényl-5 benzo[f]diazépine-1,4 et de 1,8 g de ruthénium à 5% sur charbon dans 160 cm3 de méthanol. On obtient ainsi 4,95 g d'amino-3 dihydro-2,3 oxo-2 phényl-5 (diméthyl-3,3 pipéridino) carbonylméthyl-1 1H-benzo[f]diazépine-1,4-(RS) fondant à 88°C.

La dihydro-2,3 (diméthyl-3,3 pipéridino)carbonylméthyl-1 hydroxyimino-3 oxo-2 phényl-5 1H-benzo[f]diazépine-1,4 peut être obtenue d'une manière analogue à celle décrite à l'exemple 1 pour la préparation de la dihydro-2,3 hydroxyimino-3 oxo-2 phényl-5 (pyrrolidinyl-1)carbonylméthyl-1 1H-benzo[f]diazépine-1,4, mais à partir de 16,5 g de dihydro-2,3 (diméthyl-3,3 pipéridino)carbonylméthyl-1 oxo-2 phényl-5 1H-benzo[f]diazépine-1,4, de 11,88 g de tert-butylate de potassium et de 7 cm3 de nitrite d'isopentyle dans 300 cm3 de toluène. On obtient ainsi 7 g de dihydro-2,3 (diméthyl-3,3 pipéridino)carbonylméthyl-1 hydroxyimino-3 oxo-2 phényl-5 1H-benzo[f]diazépine-1,4 fondant à 145°C.

La dihydro-2,3 (diméthyl-3,3 pipéridino)carbonylméthyl-1 oxo-2 phényl-5 1H-benzo[f]diazépine-1,4 peut être préparée de la manière suivante : une solution de 31,9 g d'acide (dihydro-2,3 oxo-2 phényl-5 1H-benzo[f]diazépine-1,4 yl-1) acétique, de 17,6 g de N,N'-diimidazole carbonyle et de 0,66 g de N,N-diméthylamino-4 pyridine dans 250 cm3 de tétrahydrofuranne est agitée pendant 18 heures à une température voisine de 20°C. On ajoute ensuite une solution de 12,3 g de diméthyl-3,3 pipéridine dans 60 cm3 de tétrahydrofuranne et agite encore pendant 5 heures et 30 minutes. Le mélange réactionnel est versé dans 500 cm3 d'eau. La phase aqueuse est extraite par 3 fois 500 cm3 puis 1 fois 250 cm3 d'oxyde de diéthyle. Les extraits organiques sont réunis, lavés par 3 fois 250 cm3 d'eau, séchés sur sulfate de magnésium et concentrés à sec sous pression réduite (2,7 kPa) à 40°C. Le résidu est purifié par chromatographie sur 700 cm3 de silice contenus dans une colonne de 6 cm de diamètre (éluants: dichlorométhane puis dichlorométhane-méthanol (98-2 en volumes)). Les fractions contenant le produit attendu sont réunies et concentrées à sec sous pression réduite (2,7 kPa). On obtient ainsi, après cristallisation dans le pentane, 15 g de dihydro-2,3 (diméthyl-3,3 pipéridino)carbonylméthyl-1 oxo-2 phényl-5 1H-benzo[f]diazépine-1,4 fondant à 86°C.

L'acide (dihydro-2,3 oxo-2 phényl-5 1H-benzo[f]diazépine-1,4 yl-1) acétique peut être préparé d'une manière analogue à celle décrite à l'exemple 1 pour la préparation de l'acide {{[dihydro-2,3 oxo-2 phényl-5 (pyrrolidinyl-1)carbonyl-méthyl-1 1H-benzo[f]diazépine-1,4 yl-3]-3 uréido}-3 phénylthio}acétique-(RS), mais à partir de 27,7 g de (dihydro-2,3 oxo-2 phényl-5 1H-benzo[f]diazépine-1,4 yl-1) acétate de tert-butyle et de 100 cm3 d'acide trifluoroacétique. On obtient ainsi 21,9 g d'acide (dihydro-2,3 oxo-2 phényl-5 1H-benzo[f]diazépine-1,4 yl-1) acétique fondant à 163°C.

Le (dihydro-2,3 oxo-2 phényl-5 1H-benzo[f]diazépine-1,4 yl-1) acétate de tert-butyle peut être préparé de la manière suivante : à une solution de 0,7 g de dihydro-2,3 oxo-2 phényl-5 1H-benzo[f]diazépine-1,4 dans 5 cm3 de tétrahydrofuranne, on ajoute, à une température voisine de 0°C, 0,99 g d'une dispersion d'hydrure de sodium à 60% dans la vaseline. Le mélange réactionnel est agité pendant 2 heures à une température voisine de 0°C puis on ajoute une solution de 0,48 cm3 de bromoacétate de tert-butyle dans 5 cm3 de tétrahydrofuranne. Le mélange réactionnel est agité 2 heures à une température voisine de 17°C puis additionné de 5 cm3 d'eau et versé dans 100 cm3 d'eau. La phase aqueuse est extraite par 4 fois 75 cm3 d'acétate d'éthyle. Les extraits organiques sont réunis , lavés par 25 cm3 d'une solution aqueuse normale de carbonate d'ammonium, séchés sur sulfate de magnésium et amenés à sec sous pression réduite (2,7 kPa) à 40°C. On obtient ainsi, après cristallisation dans le pentane, 0,99 g de (dihydro-2,3 oxo-2 phényl-5 1H-benzo[f]diazépine-1,4 yl-1) acétate de tert-butyle fondant à 149°C.

### EXEMPLE 5

En opérant comme à l'exemple 1, mais à partir de 2 g de {{[(N,N-diéthylcarbamoylméthyl)-1 dihydro-2,3 oxo-2 phényl-5 1H-benzo[f]diazépine-1,4 yl-3]-3 uréido}-3 phénylthio}acétate de tert-butyle-(RS) et de 10 cm3 d'acide trifluoroacétique et en recristallisant dans 60 cm3 d'éthanol, on obtient 1,42 g d'acide {{[(N,N-diéthyl-carbamoylméthyl)-1 dihydro-2,3 oxo-2 phényl-5 1H-benzo[f]diazépine-1,4 yl-3]-3 uréido}-3 phénylthio}acétique-(RS) fondant à 200°C en se décomposant.

Le {{[(N,N-diéthyl-carbamoylméthyl)-1 dihydro-2,3 oxo-2 phényl-5 1H-benzo[f]diazépine-1,4 yl-3]-3 uréido}-3 phénylthio}acétate de tert-butyle-(RS) peut être obtenu de la manière suivante : A une solution de 2,38 g d'(isocyanato-3 phénylthio)acétate de tert-butyle dans 10 cm3 de tétrahydrofuranne, on ajoute en 10 minutes, à une température voisine de 20°C, une solution de 3,1 g d'amino-3 (N,N-diéthyl-carbamoylméthyl)-1 dihydro-2,3 oxo-2 phényl-5 1H-benzo[f]diazépine-1,4-(RS) dans 25 cm³ de tétrahydrofuranne. Le mélange est agité pendant 18 heures à une température voisine de 20°C et dilué avec 60 cm3 d'oxyde de diéthyle. Le précipité est filtré. On obtient ainsi 3,6 g de {{[(N,N-diéthylcarbamoylméthyl)-1 dihydro-2,3 oxo-2 phényl-5 1H-benzo[f]diazépine -1,4 yl-3]-3 uréido}-3 phénylthio}acétate de tert-butyle-(RS) fondant à 220°C en se décomposant.

L'amino-3 (N,N-diéthyl-carbamoylméthyl)-1 dihydro-2,3 oxo-2 phényl-5 1H-benzo[f]diazépine-1,4-(RS) peut être obtenue de la manière suivante : Une suspension de 10,3 g de (N,N-diéthyl-carbamoylméthyl)-1 dihydro-2,3 hydroxyiminino-3 oxo-2 phényl-5 1H-benzo[f]diazépine-1,4 et de 2 g de ruthénium à 5% sur charbon dans 200 cm3 de méthanol est agitée pendant 15 heures à une température voisine de 70°C sous atmosphère d'hydrogène (800 kPa). Le catalyseur est séparé par filtration et le filtrat concentré sous pression réduite (2,7 kPa). Le résidu est purifié par chromatographie sur une colonne de 6 cm de diamètre contenant 600 cm³ de silice, en éluant d'abord avec 400 cm3 de dichlorométhane pur, puis avec 3 litres du mélange dichlorométhane-éthanol (95-5 en volumes) puis du mélange dichlorométhane-éthanol (90-10 en volumes). Les fractions comprises entre 1,25 et 5 litres sont réunies et concentrées sous pression réduite (2,7 kPa) pour donner 8,1 g de meringue blanche. Par reprise dans l'oxyde de diisopropyle, on obtient 7,6 g d'amino-3 (N,N-diéthyl-carbamoylméthyl)-1 dihydro-2,3 oxo-2 phényl-5 1H-benzo[f]diazépine-1,4-(RS) fondant à 152°C.

La (N,N-diéthyl-carbamoylméthyl)-1 dihydro-2,3 hydroxyiminino-3 oxo-2 phényl-5 1H-benzo[f]diazépine-1,4 peut être obtenue d'une manière analogue à celle décrite à l'exemple 1 pour la préparation de la dihydro-2,3 hydroxyiminino-3 oxo-2 phényl-5 (pyrrolidinyl-1)carbonylméthyl-1 1H-benzo[f]diazépine-1,4, mais à partir de 11,8 g de (N,N-diéthylcarbamoylméthyl)-1 dihydro-2,3 oxo-2 phényl-5 1H-benzo[f]diazépine-1,4, de 7,6 g de tert-butylate de potassium et de 5 cm3 de nitrite d'isopentyle dans 350 cm3 de toluène. On obtient ainsi 10,42 g de (N,N-diéthylcarbamoylméthyl)-1 dihydro-2,3 hydroxyiminino-3 oxo-2 phényl-5 1H-benzo[f]diazépine-1,4 fondant à 220°C.

La (N,N-diéthyl-carbamoylméthyl)-1 dihydro-2,3 oxo-2 phényl-5 1H-benzo[f]diazépine-1,4 peut être obtenue de la manière suivante : A une solution de 10 g de dihydro-2,3 oxo-2 phényl-5 1H-benzo[f]diazépine-1,4 et de 6,65 g de N,N-diéthyl chloro-2 acétamide dans 100 cm3 de diméthylformamide, on ajoute 0,75 g d'iodure de potassium et 17,5 g de carbonate de potassium. La suspension obtenue est agitée à une température voisine de 20°C pendant 16 heures. Le milieu réactionnel est concentré sous pression réduite (1,2 kPa). Le résidu est repris dans 100 cm3 d'acétate d'éthyle. La phase organique est lavée deux fois à l'eau puis séchée sur sulfate de magnésium et concentrée à sec sous pression rédurte (2,7 kPa). Le résidu est purifié par chromatographie sur une colonne de 6 cm de diamètre contenant 900 cm3 de silice, en éluant d'abord avec le mélange cyclohexane-acétate d'éthyle (50-50 en volumes) puis à l'acétate d'éthyle pur et en recueillant des fractions de 125 cm3. Les fractions comprises entre 3,75 et 4,75 litres sont réunies et évaporées à sec pour donner 11,9 g de (N,N-diéthyl-carbamoylméthyl)-1 dihydro-2,3 oxo-2 phényl-5 1H-benzo[f]diazépine-1,4 sous forme d'une meringue utilisée telle quelle dans les synthèses ultérieures.

### EXEMPLE 6

On chauffe 2 minutes à une température voisine de 100°C une solution de 2,5 g de N-(dihydro-2,3 méthyl-1 oxo-2 phényl-5 1H-benzo[f]diazépine-1,4 yl-3) N'-(oxo-1 indanyl-6) urée-(RS) et de 6,12 g de chlorhydrate d'hydroxylamine dans 80 cm3 de pyridine aqueuse (75-25 en volumes). Le milieu réactionnel est refroidi à une température voisine de 20°C et concentré sous pression réduite (2,7 kPa). On dilue avec 200 cm3 d'eau, acidifie à pH 1 avec une solution aqueuse d'acide chlorhydrique 6N et extrait par 200 cm3 d'acétate d'éthyle. La phase organique est lavée par deux fois 200 cm3 d'eau, séchée sur sulfate de magnésium, puis concentrée à sec. Le résidu est purifié par chromatographie sous pression (4,1 MPa) sur une colonne de 300 g de silice 15-20 microns, en éluant d'abord avec 3 litres du mélange dichlorométhane-méthanol (98/2 en volumes), puis 850 cm3 du mélange dichlorométhane-méthanol (95/5 en volumes). Les fractions comprises entre 1750 et 3850 cm3 sont réunies et évaporées à sec pour donner 1,7 g d'une meringue blanche. Après recristallisation dans 30 cm3 d'acétate d'éthyle, on obtient 1 g de N-(dihydro-2,3 méthyl-1 oxo-2 phényl-5 1H-benzo[f]diazépine-1,4 yl-3) N'-(hydroxyimino-1 indanyl-6) urée-(E)-(RS) fondant à 218°C.

La N-(dihydro-2,3 méthyl-1 oxo-2 phényl-5 1H-benzo[f]diazépine-1,4 yl-3) N'-(oxo-1 indanyl-6) urée-(RS) peut être obtenue de la manière suivante : A une solution de 2,5 g d'amino-3 méthyl-1 oxo-2 phényl-5 benzo[f]diazépine-1,4 dans 90 cm3 de tétrahydrofuranne, on ajoute en 10 minutes une solution de 1,9 g d'isocyanato-6 indanone-1 dans 20 cm3 de tétrahydrofuranne. Après 15 minutes d'agitation à une température voisine de 20°C, on filtre l'insoluble et le lave par deux fois 15 cm3 de tétrahydrofuranne puis par deux fois 50 cm3 d'oxyde de diisopropyle, pour obtenir 3,2 g de N-(dihydro-2,3 méthyl-1 oxo-2 phényl-5 1H-benzo[f]diazépine-1,4 yl-3) N'-(oxo-1 indanyl-6) urée-(RS) fondant vers 270°C en se décomposant.

L'isocyanato-6 indanone-1 peut être préparée de la manière suivante : A une suspension refroidie à environ -20°C, de 1,6 g d'amino-6 indanone-1 et de 0,2 g de noir animal dans 30 cm3 de toluène, on ajoute en 25 minutes, une solution de 1,6 g de carbonate de bis(trichlorométhyle) dans 20 cm3 de toluène, à une température voisine de -20°C. On laisse réchauffer à température ambiante, puis on chauffe 2 heures à une température voisine de 110°C. La suspension obtenue est refroidie à une température voisine de 20°C, filtrée et le filtrat est concentré sous pression réduite (2,7 kPa) pour donner 1,9 g d'isocyanato-6 indanone-1 fondant à 72°C.

L'amino-6 indanone-1 peut être préparée selon la méthode décrite par C.K. INGOLD et H.A. PIGGOT, J. Chem. Soc., 123, 1469 (1923).

### EXEMPLE 7

A une solution de 1,9 g de {dihydro-3,4 [(dihydro-2,3 méthyl-1 oxo-2 phényl-5 1H-benzo[f]diazépine-1,4 yl-3)-3 uréido]-6 2H-benzopyrannylidène-4}-2 acétate d'éthyle-(E)-(RS) dans 40 cm3 de pyridine, on ajoute 70 cm3 d'une solution aqueuse de soude 0,1 N, puis chauffe à une température voisine de 100°C pendant 2 heures. Après refroidissement à une température voisine de 20°C, on concentre sous pression réduite (1,2 kPa). Le résidu est dilué avec 100 cm3 d'eau et lavé par 100 cm3 d'oxyde de diéthyle. La phase aqueuse est amenée à pH 1 avec une solution aqueuse d'acide chlorhydrique 4N et extraite par 100 cm3 d'acétate d'éthyle. La phase organique est lavée avec 200 cm3 d'eau, séchée sur sulfate de magnésium et concentrée à sec. Le résidu obtenu est purifié par chromatographie sur une colonne de 2,5 cm de diamètre contenant 160 cm3 de silice, en éluant avec 500 cm3 de dichlorométhane, puis 2 litres du mélange dichlorométhane-méthanol (98-2 en volumes), puis 1 litre du mélange dichlorométhane-méthanol (95-5 en volumes), puis 2 litres du mélange dichlorométhane-méthanol (93-7 en volumes) et en recueillant des fractions de 250 cm3. Les fractions comprises entre 1 et 5,5 litres sont réunies et evaporées à sec, pour donner 0,33 g d'un solide jaune. Par recristallition dans 25 cm3 d'oxyde de diisopropyle, on obtient 0,26 g d'acide {dihydro-3,4 [(dihydro-2,3 méthyl-1 oxo-2 phényl-5 1H-benzo[f]diazépine-1,4 yl-3)-3 uréido]-6 2H-benzopyrannylidène-4}-2 acétique-(E)-(RS) fondant à 250°C.

La {dihydro-3,4 [(dihydro-2,3 méthyl-1 oxo-2 phényl-5 1H-benzo[f]diazépine-1,4 yl-3)-3 uréido]-6 2H-benzopyrannylidène-4}-2 acétate d'éthyle-(E)-(RS) peut être préparée de la manière suivante : A une solution de 2 g d'amino-3 méthyl-1 oxo-2 phényl-5 benzo[f]diazépine-1,4-(RS) dans 25 cm3 de tétrahydrofuranne, on ajoute en 10 minutes une solution de 2,1 g de (dihydro-3,4 isocyanato-6 2H-benzopyrannylidène-4)-2 acétate d'éthyle-(E) dans 20 cm3 de tétrahydrofuranne. Après 1 heure d'agitation à une température voisine de 20°C, le milieu réactionnel est concentré sous pression réduite (2,7 kPa) et repris dans 50 cm3 d'acétate d'éthyle. La phase organique est lavée avec 50 cm3 d'eau, séchée sur sulfate de magnésium puis évaporée à sec pour donner 3,4 g de produit sous forme d'une meringue ocre. Par cristallisation dans 40 cm3 d'acétate d'éthyle, on obtient 2,4 g de {dihydro-3,4 [(dihydro-2,3 méthyl-1 oxo-2 phényl-5 1H-benzo[f]diazépine-1,4 yl-3)-3 uréido]-6 2H-benzopyrannylidène-4}-2 acétate d'éthyle-(E)-(RS) fondant à 230°C.

Le (dihydro-3,4 isocyanato-6 2H-benzopyrannylidène-4)-2 acétate d'éthyle-(E) peut être obtenu de la manière suivante : A une suspension de 2,4 g d'(amino-6 dihydro-3,4 2H-benzopyrannylidène-4)-2 acétate d'éthyle-(E) et de 0,1 g de noir animal dans 45 cm3 de toluène, refroidie à une température voisine de -20°C, on ajoute en 10 minutes une solution de 1,52 g de carbonate de bis(trichlorométhyle) dans 20 cm3 de toluène. Après 10 minutes d'agitation à une température voisine de -20°C, on porte 1 heure à une température voisine de 110°C avant de refroidir à une température voisine de 20°C. On filtre sur célite et concentre le filtrat à sec sous pression réduite (2,7 kPa) pour obtenir 2,7 g de (dihydro-3,4 isocyanato-6 2H-benzopyrannylidène-4)-2 acétate d'éthyle-(E) sous la forme d'une huile utilisée telle quelle dans les synthèses ultérieures.

L'(amino-6 dihydro-3,4 2H-benzopyrannylidène-4)-2 acétate d'éthyle-(E) peut être préparé de la manière suivante : Une suspension de 3,4 g de (nitro-6 dihydro-3,4 2H-benzopyrannylidène-4)-2 acétate d'éthyle-(E) et de 2,7 g de fer en poudre dans 90 cm3 d'éthanol aqueux à 50% est additionnée de 2,7 cm3 d'une solution aqueuse d'acide chlorhydrique 12N. On chauffe à une température voisine de 80°C pendant 1 heure puis refroidit à une température voisine de 50°C. On filtre sur célite et lave avec 20 cm3 d'éthanol puis 20 cm3 d'eau puis évapore sous pression réduite (2,7 kPa). On acidifie à pH 1 avec 25 cm3 d'une solution d'acide chlorhydrique N et lave avec 100 cm3 d'oxyde de diéthyle. On alcalinise la phase aqueuse à pH 8 avec de l'hydrogénocarbonate de sodium et extrait avec 200 cm3 d'acétate d'éthyle. On sèche sur sulfate de magnésium et évapore le filtrat pour obtenir 2,5 g d'(amino-6 dihydro-3,4 2H-benzopyrannylidène-4)-2 acétate d'éthyle-(E) fondant à 97°C.

Le (nitro-6 dihydro-3,4 2H-benzopyrannylidène-4)-2 acétate d'éthyle-(E) peut être obtenu de la manière suivante : Dans un appareillage purgé à l'argon, on introduit 9,9 g d'une suspension à 60% d'hydrure de sodium dans l'huile. L'huile est éliminée par deux lavages avec 100 cm3 d'hexane. On ajoute 200 cm3 de tétrahydrofuranne, puis, à une température voisine de 20°C, on coule en 30 minutes, 62 cm3 de phosphonoacétate de triéthyle. Après 10 minutes d'agitation à une température voisine de 20°C, on coule 12 g de dihydro-3,4 nitro-6 one-4 2H-benzopyranne. Après 1 heure d'agitation à une température voisine de 20°C, on concentre le milieu réactionnel sous pression réduite (2,7 kPa), dilue avec 500 cm3 d'eau et extrait par deux fois 200 cm3 d'acétate d'éthyle. Les phases organiques sont rassemblées et lavées par 500 cm3 d'eau, séchées sur sulfate de magnésium et évaporées à sec pour donner une huile qui est chromatographiée sur une colonne de 6 cm de diamètre contenant 800 cm3 de silice, en éluant avec le mélange cyclohexane-acétate d'éthyle (93-7 en volumes) et en recueillant des fractions de 200 cm3. Les fractions comprises entre 1 et 2,2 litres sont rassemblées et évaporées à sec pour donner 3,5 g de (nitro-6 dihydro-3,4 2H-benzopyrannylidène-4)-2 acétate d'éthyle-(E) fondant à 135°C.

La dihydro-3,4 nitro-6 2H-benzopyranone-4 peut être obtenue par la méthode décrite par C.D. HURD et S. HAYAO, J. Amer. Chem. Soc., 76, 5065 (1954).

### EXEMPLE 8

Une solution de 3,5 g de {[dihydro-2,3 oxo-2 phényl-5 (pyrrolidinyl-1)carbonylméthyl-1 1H-benzo[f]diazépine-1,4 yl-3]-3 uréido}-3 phénylméthylènaminooxy-2 acétate de tert-butyle-(E)-(RS) dans 15 cm³ d'acide trifluoroacétique est agitée 2 heures à une température voisine de 20°C. On concentre le milieu réactionnel sous pression réduite (2,7 kPa). Le résidu est repris dans 50 cm3 d'eau et extrait par 50 cm3 d'acétate d'éthyle. On sèche la phase organique sur sulfate de magnésium et évapore à sec pour obtenir 3 g d'huile jaune. Par cristallisation dans 40 cm3 d'acétate d'isopropyle, on obtient 1,6 g d'acide {[dihydro-2,3 oxo-2 phényl-5 (pyrrolidinyl-1)carbonylméthyl-1 1H-benzo[f]diazépine-1,4 yl-3]-3 uréido}-3 phénylméthylènaminooxy-2 acétique-(E)-(RS) fondant à 175°C.

Le {[dihydro-2,3 oxo-2 phényl-5 (pyrrolidinyl-1)carbonylméthyl-1 1H-benzo[f]diazépine-1,4 yl-3]-3 uréido}-3 phénylméthylènaminooxy-2 acétate de tert-butyle-(E)-(RS) peut être obtenu de la manière suivante : A une solution de 2,8 g d'amino-3 dihydro-2,3 oxo-2 phényl-5 (pyrrolidinyl-1)carbonylméthyl-1 1H-benzo[f]diazépine-1,4-(RS) dans 30 cm3 de tétrahydrofuranne, on ajoute en 10 minutes une solution de 2,35 g d'isocyanato-3 phénylméthylènaminooxyacétate de tert-butyle-(E), dans 25 cm3 de tétrahydrofuranne et garde 1 heure à une température voisine de 20°C. Le milieu réactionnel est évaporé à sec sous pression réduite (2,7 kPa). Le résidu est purifié par chromatographie sur une colonne de 4 cm de diamètre contenant 400 cm3 de silice, en éluant d'abord avec 500 cm3 du mélange acétate d'éthyle-cyclohexane (40-60 en volumes), puis avec 500 cm3 du mélange acétate d'éthyle-cyclohexane (50-50 en volumes), puis avec 1 litre du mélange acétate d'éthyle-cyclohexane (60-40 en volumes), puis avec 500 cm3 du mélange acétate d'éthyle-cyclohexane (80-20 en volumes), puis avec de l'acétate d'éthyle pur et en recueillant des fractions de 200 cm3. Les fractions comprises entre 2,2 et 3 litres sont réunies et évaporées à sec pour donner 3,5 g de {[dihydro-2,3 oxo-2 phényl-5 (pyrrolidinyl-1)carbonylméthyl-1 1H-benzodiazépine-1,4 yl-3] -3 uréido}-3 phénylméthylènaminooxy-2 acétate de tert-butyle-(E)-(RS), sous la forme d'une meringue utilisée telle quelle dans les synthèses ultérieures.

L'isocyanato-3 phénylméthylènaminooxyacétate de tert-butyle-(E) peut être obtenu de la manière suivante : A une solution de 6,2 g d'amino-3 phénylméthylènaminooxyacétate de tert-butyle-(E) dans 100 cm3 de toluène, on ajoute 0,6 g de noir animal, on refroidit cette suspension à une température voisine de -30°C, puis ajoute en 10 minutes 3 cm3 de chloroformiate de trichlorométhyle dans 5 cm3 de toluène et laisse réchauffer à une température voisine de 20°C avant de chauffer 2 heures à une température voisine de 110°C. On refroidit à une température voisine de 20°C, filtre sur célite. Le précipité est lavé par 25 cm3 de toluène et les phases toluéniques sont rassemblées et concentrées à sec (2,7 kPa). On obtient ainsi 6,8 g d'isocyanato-3 phénylméthylènaminooxyacétate de tert-butyle-(E), sous forme d'une huile utilisée telle quelle dans les synthèses ultérieures.

L'amino-3 phénylméthylènaminooxyacêtate de tert-butyle-(E) peut être obtenu de la manière suivante : A une solution de 7,5 g de nitro-3 phénylméthylènaminooxyacétate de tert-butyle-(E) dans 100 cm3 d'éthanol, on ajoute 0,75 g de dioxyde de platine. La suspension est agitée 45 minutes à une température voisine de 20°C sous atmosphère d'hydrogène (128 kPa).

Le catalyseur est séparé par filtration et le filtrat est concentré sous pression réduite (2,7 kPa). Le résidu est repris dans 50 cm3 d'eau et extrait avec 50 cm3 d'acétate d'éthyle. Après séchage sur sulfate de magnésium, on évapore le filtrat à sec pour obtenir 6,2 g d'amino-3 phénylméthylènaminooxyacétate de tert-butyle-(E) sous la forme d'une huile utilisée telle quelle dans les synthèses ultérieures.

Le nitro-3 phénylméthylènaminooxyacétate de tert-butyle-(E) peut être obtenu de la manière suivante : En opérant sous atmosphère d'argon, 1,3 g d'une dispersion d'hydrure de sodium à 60% dans l'huile sont lavés par deux fois 50 cm3 d'hexane. On ajoute 100 cm3 de tétrahydrofuranne puis on refroidit à une température voisine de 5°C. On ajoute en 15 minutes une solution de 4,98 g de nitro-3 benzaldoxime-(E) dans 20 cm3 de tétrahydrofuranne puis agite 1 heure à une température voisine de 20°C. On coule alors en 15 minutes 5 cm3 de bromo-2 acétate de tert-butyle et agite à une température voisine de 25°C. On coule 200 cm3 d'eau et 100 cm3 d'acétate d'éthyle. On décante et lave la phase organique avec 100 cm3 d'eau. On sèche les phases organiques sur sulfate de magnésium et concentre le filtrat à sec (2,7 kPa) pour obtenir le nitro-3 phénylméthylènaminooxyacétate de tert-butyle-(E) fondant à 110°C.

Le nitro-3 benzaldoxime-(E) peut être préparé par la méthode décrite par S. GABRIEL, Ber., 16, 1997 (1883).

### EXEMPLE 9

Une suspension de 1,3 g d'un mélange d'isomères Z et E (50:50) de l'acide {{{[dihydro-2,3 oxo-2 phényl-5 (pyrrolidinyl-1)carbonylméthyl-1 1H-benzo[f]diazépine-1,4 yl-3]-3 uréido}-3 phényl}-1 éthoxycarbonyl-1 méthylène}aminooxy-2 acétique-(RS) dans 15 cm3 d'éthanol, 3,8 cm3 de soude 1N et de 5 cm3 d'eau, est agitée pendant 16 heures à une température voisine de 20°C. Le milieu réactionnel est concentré sous pression réduite (2,7 kPa) et dilué avec 50 cm3 d'eau. La phase aqueuse est lavée avec 30 cm3 d'oxyde de diéthyle puis acidifiée à pH 1 avec 5 cm3 d'une solution aqueuse d'acide chlorhydrique 1N. On extrait la phase aqueuse par 50 cm3 d'acétate d'éthyle, sèche sur sulfate de magnésium et concentre sous pression réduite à un volume de 10 cm3 avant de diluer avec 40 cm3 d'oxyde de diisopropyle. Par filtration on obtient 0,63 g d'un mélange d'isomères Z et E (50:50) d'acide {{[(dihydro-2,3 oxo-2 phényl-5 (pyrrolidinyl-1)carbonylméthyl-1 1H-benzo[f]diazépine-1,4 yl-3]-3 uréido}-3 phényl}-2 (carboxyméthyloxyimino)-2 acétique-(RS) fondant à 190°C.

Le mélange d'isomères Z et E (50:50) de l'acide {{{[dihydro-2,3 oxo-2 phényl-5 (pyrrolidinyl-1)carbonylméthyl-1 1H-benzo[f]diazépine-1,4 yl-3]-3 uréido}-3 phényl}-1 éthoxycarbonyl-1 méthylène}aminooxy-2 acétique-(RS) peut être obtenu de la manière suivante : Une solution de 1,45 g d'un mélange d'isomères Z et E (50:50) de {{[(dihydro-2,3 oxo-2 phényl-5 (pyrrolidinyl-1)carbonylméthyl-1 1H-benzo[f]diazépine-1,4 yl-3]-3 uréido}-3 phényl}-2 (tert-butoxycarbonylméthyloxyimino)-2 acétate d'éthyle-(RS) dans 30 cm3 d'acide trifluoroacétique est agitée 2 heures à une température voisine de 20°C. On concentre le milieu réactionnel sous pression réduite (2,7 kPa). Le résidu est repris dans 50 cm3 d'oxyde de diisopropyle pour donner 1,35 g d'un solide blanc. Ce solide est dissous en présence de 1 g d'hydrogénocarbonate de sodium dans 50 cm3 d'eau et la phase organique est rapidement lavée par 50 cm3 d'acétate d'éthyle. La phase aqueuse est acidifiée à pH=1 avec une solution aqueuse d'acide chlorhydrique 1N. Par extraction avec 100 cm3 d'acétate d'éthyle, séchage sur sulfate de magnésium et concentration à sec, on obtient 1,3 g d'un mélange d'isomères Z et E (50:50) de l'acide {{{[dihydro-2,3 oxo-2 phényl-5 (pyrrolidinyl-1)carbonylméthyl-1 1H-benzo[f]diazépine-1,4 yl-3]-3 uréido}-3 phényl}-1 éthoxycarbonyl-1 méthylène}aminooxy-2 acétique-(RS) fondant vers 150°C.

Le mélange d'isomères Z et E (50:50) de {{[(dihydro-2,3 oxo-2 phényl-5 (pyrrolidinyl-1)carbonylméthyl-1 1H-benzo[f]diazépine-1,4 yl-3]-3 uréido}-3 phényl}-2 (tert-butoxycarbonylméthyloxyimino)-2 acétate d'éthyle-(RS) peut être obtenu de la manière suivante : A une solution de 1,45 g d'amino-3 dihydro-2,3 oxo-2 phényl-5 (pyrrolidinyl-1)carbonylméthyl-1 1H-benzo[f]diazépine-1,4 dans 20 cm3 de tétrahydrofuranne, on ajoute en 5 minutes 1,55 g d'un mélange d'isomères Z et E de l'isocyanato-3 alpha-tert-butoxycarbonylméthyloxyimino phénylacétate d'éthyle dans 10 cm3 de tétrahydrofuranne et agite 3 heures à une température voisine de 20°C. On verse sur 50 cm3 d'eau et extrait par 100 cm3 d'acétate d'éthyle. On sèche sur sulfate de magnésium et concentre sous pression réduite (2,7 kPa). Le résidu (2,2 g) est purifié par chromatographie sur une colonne de 3,5 cm de diamètre contenant 200 cm3 de silice, en éluant avec le mélange dichlorométhane-méthanol (97-3 en volumes) et en recueillant des fractions de 50 cm3. Les fractions comprises entre 250 et 400 cm3 sont réunies et évaporées à sec pour donner 1,45 g d'un mélange d'isomères Z et E (50:50) de {{[(dihydro-2,3 oxo-2 phényl-5 (pyrrolidinyl-1)carbonylméthyl-1 1H-benzo[f]diazépine-1,4 yl-3]-3 uréido}-3 phényl}-2 (tert-butoxycarbonylméthyloxyimino)-2 acétate d'éthyle-(RS) sous forme d'une meringue utilisée telle quelle dans les synthèses ultérieures.

Le mélange d'isomères Z et E de l'isocyanato-3 alpha-tert-butoxycarbonylméthyloxyimino phénylacétate d'éthyle peut être obtenu de la manière suivante : A une suspension de 1,4 g d'amino-3 alpha-tert-butoxycarbonylméthyloxyimino phénylacétate d'éthyle-(Z) et de 0,05 g de noir animal dans 30 cm3 de toluène sec refroidie à une température voisine de -20°C, on ajoute en 10 minutes une solution de 0,67 g de carbonate de bis(trichlorométhyle) dans 15 cm3 de toluène sec. On laisse réchauffer à une température voisine de 20°C puis porte 1 heure à une température voisine de 110°C. On refroidit à une température voisine de 20°C, filtre sur célite, lave par 5 cm3 de toluène sec et concentre à sec sous pression réduite (1,2 kPa), pour obtenir 1,55 g d'un mélange des isomères Z et E de l'isocyanato-3 alpha-tert-butoxycarbonylméthyloxyimino phénylacétate d'éthyle sous forme d'une huile utilisée telle quelle dans les synthèses ultérieures.

L'amino-3 alpha-tert-butoxycarbonylméthyloxyimino phénylacétate d'éthyle-(Z) peut être obtenu de la manière suivante : A une solution de 2,61 g de nitro-3 alpha-tert-butoxycarbonylméthyloxyimino phénylacétate d'éthyle-(Z) dans 70 cm3 d'éthanol, on ajoute 0,15 g de dioxyde de platine. La suspension est agitée 40 minutes à une température voisine de 20°C sous atmosphère d'hydrogène (128 kPa). Le catalyseur est séparé par filtration et le filtrat est concentré à sec sous pression réduite (2,7 kPa) pour donner 2,3 g d'amino-3 alpha-tert-butoxycarbonylméthyloxyimino phénylacétate d'éthyle-(Z) sous forme d'une huile utilisée telle quelle dans les synthèses ultérieures.

Le nitro-3 alpha-tert-butoxycarbonylméthyloxyimino phénylacétate d'éthyle-(Z) peut être obtenu de la manière suivante : Dans un appareillage purgé à l'argon, on introduit 0,46 g d'une dispersion à 60% d'hydrure de sodium dans l'huile. L'huile est éliminée par deux lavages avec 40 cm³ d'heptane. On ajoute 30 cm3 de tétrahydrofuranne, puis 2,5 g de nitro-3 alpha-hydroxyimino phénylacétate d'éthyle-(Z) en 15 minutes à une température voisine de 20°C. Après 10 minutes, on ajoute en 10 minutes 1,8 cm3 de bromo-2 acétate de tert-butyle dans 10 cm3 de tétrahydrofuranne. Après 1 heure d'agitation à une température voisine de 20°C, on dilue avec 100 cm3 d'acétate d'éthyle et lave avec 100 puis 50 cm3 d'eau, sèche sur sulfate de magnésium, titre et concentre à sec sous pression réduite (2,7 kPa) pour obtenir 3 g de nitro-3 alpha-tert-butoxycarbonylméthyloxyimino phénylacétate d'éthyle-(Z), sous forme d'une huile utilisée telle quelle dans les synthèses ultérieures.

Le nitro-3 alpha-hydroxyimino phénylacétate d'éthyle-(Z) peut être obtenu de la manière suivante : A une solution de 10 g de nitro-3 phénylglyoxylate d'éthyle dans 250 cm3 de pyridine, on ajoute une solution de 62 g de chlorhydrate d'hydroxylamine dans 200 cm3 d'eau et porte 2 heures à une température voisine de 100°C. Le milieu réactionnel est concentré sous pression réduite (1,2 kPa). Le résidu est dilué avec 250 cm3 d'eau et extrait avec 250 cm3 d'acétate d'éthyle. La phase organique est lavée avec une solution aqueuse d'acide chlorhydrique 1N, séchée sur sulfate de magnésium, filtrée et évaporée à sec pour donner un solide blanc. Ce solide est purifié par chromatographie sur une colonne de 4 cm de diamètre contenant 500 cm3 de silice en éluant avec le mélange cyclohexane-acétate d'éthyle et en recueillant des fractions de 100 cm3. Les fractions comprises entre 1 et 1,2 litres sont jointes et concentrées à sec pour donner 1,8 g de nitro-3 alpha-hydroxyimino phénylacétate d'éthyle-(E), sous forme d'une huile incolore ; les fractions comprises entre 1,4 et 1,6 litres sont jointes et concentrées à sec pour donner 2,5 g de nitro-3 alpha-hydroxyimino phénylacétate d'éthyle-(Z), sous forme d'un solide utilisé tel quel dans les synthèses ultérieures.

Le nitro-3 phénylglyoxylate d'éthyle peut être préparé par la méthode décrite par E. ADLEROVA, P. VEJDELKOVA et M. PROTIVA, Collection Czech. Chem. Commun., 29, 97-120 (1964).

### EXEMPLE 10

A une solution de 1,1 g d'(amino-3 benzyl)-5 diméthyl-2,2 dioxanne-1,3 dione-4,6 dans 20 cm3 de tétrahydrofuranne sec, on ajoute en 10 minutes une solution de 2 g de dihydro-2,3 isocyanato-3 oxo-2 phényl-5 (pyrrolidinyl-1)carbonylméthyl-1 1H-benzo[f]diazépine-1,4-(RS) dans 15 cm3 de tétrahydrofuranne sec. Après 1 heure d'agitation à une température voisine de 20°C, on coule 100 cm3 d'acétate d'éthyle et l'on extrait la phase organique par 50 cm3 d'eau puis 35 cm3 d'une solution aqueuse d'hydrogénocarbonate de sodium à 5%. Les phases aqueuses sont réunies et acidifiées à pH 1 avec une solution aqueuse d'acide chlorhydrique 2N puis extraites par 100 cm3 d'acétate d'éthyle. La phase organique est séchée sur sulfate de magnésium, filtrée et évaporée à sec sous pression réduite (2,7 kPa). Le résidu est purifié par chromatographie sous pression (4,1 MPa) sur une colonne contenant 300 g de silice 55-105 microns, en éluant avec le mélange dichlorométhane-méthanol (97-3 en volumes). Les fractions comprises entre 1850 et 2500 cm3 sont réunies et évaporées à sec (2,7 kPa) pour donner 1,3 g d'un résidu qui est repris dans 30 cm3 d'oxyde de diéthyle. On obtient ainsi 1 g de {{[dihydro-2,3 oxo-2 phényl-5 (pyrrolidinyl-1)carbonylméthyl-1 1H-benzo[f]diazépine-1,4 yl-3]-3 uréido}-3 benzyl}-5 diméthyl-2,2 dioxanne-1,3 dione-4,6-(RS) fondant à 190°C.

La dihydro-2,3 isocyanato-3 oxo-2 phényl-5 (pyrrolidinyl-1)carbonylméthyl-1 1H-benzo[f]diazépine-1,4-(RS) peut être obtenue de la manière suivante : A une suspension de 2 g d'amino-3 dihydro-2,3 oxo-2 phényl-5 (pyrrolidinyl-1)carbonylméthyl-1 1H-benzo[f]diazépine-1,4-(RS) et de 0,03 g de noir animal dans 30 cm3 de toluène sec refroidie à une température voisine de -30°C, on ajoute en 15 minutes une solution de 0,82 g de carbonate de bis(trichlorométhyle) dans 15 cm3 de toluène. Après 15 minutes d'agitation à une température voisine de -30°C, on laisse revenir à une température voisine de 20°C, puis porte à une température voisine de 110°C pendant 40 minutes. Après refroidissement à une température voisine de 20°C, on filtre sur célite, rince par 5 cm3 de toluène sec et concentre sous pression réduite (1,2 kPa) pour obtenir 2,1 g de dihydro-2,3 isocyanato-3 oxo-2 phényl-5 (pyrrolidinyl-1)carbonylméthyl-1 1H-benzo[f]diazépine-1,4-(RS), sous la forme d'une meringue utilisée telle quelle dans les synthèses ultérieures.

L'(amino-3 benzyl)-5 diméthyl-2,2 dioxanne-1,3 dione-4,6 peut être obtenue de la manière suivante : Une suspension de 7 g de (nitro-3 benzylidène)-5 diméthyl-2,2 dioxanne-1,3 dione-4,6 et de 0,2 g de palladium à 10% sur charbon dans 300 cm3 d'éthanol est agitée 40 minutes à une température voisine de 20°C sous atmosphère d'hydrogène (128 kPa). Le catalyseur est séparé par filtration et le filtrat est concentré à sec sous pression réduite (2,7 kPa) pour donner 6,4 g de résidu. Ce résidu est purifié par chromatographie sur une colonne de 4 cm de diamètre contenant 200 cm3 de silice en éluant avec le mélange acétate d'éthyle-cyclohexane (60-40 en volumes) et en recueillant des fractions de 60 cm3. Les fractions comprises entre 180 et 360 cm3 sont jointes et concentrées à sec pour donner 3,2 g d'une huile qui est de nouveau purifiée par chromatographie sous pression (2,1 MPa) sur une colonne contenant 300 g de silice 55-105 microns, en éluant avec le mélange cyclohexane-acétate d'éthyle (60-40 en volumes). Les fractions comprises entre 1850 et 2550 cm3 sont réunies et évaporées à sec (2,7 kPa) pour donner 1,3 g d'(amino-3 benzyl)-5 diméthyl-2,2 dioxanne-1,3 dione-4,6 sous la forme d'une huile utilisée telle quelle dans les synthèses ultérieures.

La (nitro-3 benzylidène)-5 diméthyl-2,2 dioxanne-1,3 dione-4,6 peut être obtenue de la manière suivante : Une solution de 30,2 g de nitro-3 benzaldéhyde et de diméthyl-2,2 dioxanne-1,3 dione-4,6 dans 400 cm3 de diméthylformamide est agitée 18 heures à une température voisine de 20°C. Le milieu réactionnel est dilué avec 500 cm3 d'acétate d'éthyle, lavé cinq fois à l'eau, séché sur sulfate de magnésium, filtré et concentré à sec (1,2 kPa) pour donner une huile. Cette huile est cristallisée dans 100 cm3 d'éthanol pour donner 26 g de (nitro-3 benzylidène)-5 diméthyl-2,2 dioxanne-1,3 dione-4,6 sous forme d'un solide fondant à 128°C.

### EXEMPLE 11

A une solution de 1,7 g de (N,N-diéthyl-carbamoylméthyl)-1 dihydro-2,3 isocyanato-3 oxo-2 phényl-5 1H-benzo[f]diazépine-1,4-(RS) et de 0,05 g de N,N-diméthylamino-4 pyridine dans 30 cm3 de tétrahydrofuranne, on ajoute en 5 minutes une solution de 1,3 g d'(amino-3 phényl)-2 éthylènesulfonate de tétrabutylammonium-(E) dans 30 cm3 de dichlorométhane. Après 18 heures d'agitation à une température voisine de 20°C, on chauffe 2 heures et 30 minutes à une température voisine de 60°C. Le milieu réactionnel est concentré sous pression réduite (2,7 kPa). Le résidu est chromatographié sur une colonne de 3 cm de diamètre contenant 200 cm3 de silice en éluant avec le mélange dichlorométhane-éthanol (100-0, puis 95-5, puis 90-10, puis 80-20 en volumes). Les fractions contenant le produit attendu sont jointes et évaporées à sec sous pression réduite pour donner 1,37 g de {{[N,N-diéthylcarbamoylméthyl)-1 dihydro-2,3 oxo-2 phényl-5 1H-benzo[f]diazépine-1,4 yl-3]-3 uréido}-3 phényl}-2 éthylènesulfonate de tétrabutylammonium-(E)-(RS) sous forme d'une meringue brune. Ce sel de tétrabutylammonium est agité en présence de 10 g de résine acidesulfonique (IRN-77) dans 50 cm3 de méthanol, pendant 16 heures, à une température voisine de 20°C. La résine est filtrée et lavée par trois fois 20 cm3 de méthanol. Le filtrat est agité en présence de 0,25 g de noir animal, filtré et évaporé à sec sous pression réduite (2,7 kPa). Le résidu est dissous dans 5 cm3 de méthanol, dilué avec 40 cm3 d'éthanol puis refroidi 16 heures à une température de +5°C. On élimine ainsi 0,22 g d'un solide. Le filtrat est dilué avec 50 cm3 d'oxyde d'éthyle pour obtenir 0,31 g d'acide {{[(N,N-diéthyl-carbamoylméthyl)-1 dihydro-2,3 oxo-2 phényl-5 1H-benzo[f]diazépine-1,4 yl-3]-3 uréido}-3 phényl}-2 éthylènesulfonique-(E)-(RS) fondant à 250°C en se décomposant.

La (N,N-diéthyl-carbamoylméthyl)-1 dihydro-2,3 isocyanato-3 oxo-2 phényl-5 1H-benzo[f]diazépine-1,4-(RS) peut être obtenue comme à l'exemple 10, pour la préparation de la dihydro-2,3 isocyanato-3 oxo-2 phényl-5 (pyrrolidinyl-1)carbonylméthyl-1 1H-benzo[f]diazépine-1,4-(RS), mais à partir de 1,6 g d'amino-3 (N,N-diéthyl-carbamoylméthyl)-1 dihydro-2,3 oxo-2 phényl-5 1H-benzo[f]diazépine-1,4-(RS), de 0,89 g de carbonate de bis(trichlorométhyle) et de 0,1 g de noir animal dans 25 cm3 de toluène. On obtient ainsi 1,71 g de (N,N-diéthyl-carbamoylméthyl)-1 dihydro-2,3 isocyanato-3 oxo-2 phényl-5 1H-benzo[f]diazépine-1,4-(RS) sous forme d'une meringue utilisée telle quelle dans les synthèses ultérieures.

L'(amino-3 phényl)-2 éthylènesulfonate de tétrabutylammonium-(E) peut être obtenu de la manière suivante : Une solution de (nitro-3 phényl)-2 éthylènesulfonate de tétrabutylammonium-(E) dans 30 cm3 d'eau et 30 cm3 d'une solution à 20% de sulfure d'ammonium est agitée pendant 21 heures à une température voisine de 20°C, puis chauffée 2 heures et 30 minutes à une température voisine de 60°C puis chauffée 3,5 heures à une température voisine de 80°C. Le milieu réactionnel est concentré à sec sous pression réduite (1,2 kPa) et le résidu repris dans 60 cm3 d'eau, filtré et lavé par deux fois 20 cm3 d'eau, pour éliminer un insoluble brun. Le filtrat est extrait par 7 fois 100 cm3 de dichlorométhane. On ajoute 10 g de dihydrogénophosphate de sodium, puis réextrait par 3 fois 100 cm3 de dichlorométhane. Les phases chlorométhyléniques sont réunies, séchées sur sulfate de magnésium et concentrées à sec pour donner 4,48 g d'(amino-3 phényl)-2 éthylène sulfonate de tétrabutylammonium-(E), sous forme d'une huile utilisée telle quelle dans les synthèses ultérieures.

Le (nitro-3 phényl)-2 éthylènesulfonate de tétrabutylammonium-(E) peut être obtenu de la manière suivante : Une suspension de 13,08 g de (nitro-3 phényl)-2 éthylènesulfonate d'éthyle-(E), dans 25 cm3 d'acide sulfurique à 95% et 4 cm3 d'eau est portée pendant 5,5 heures à une température voisine de 60°C. Le milieu réactionnel est refroidi à une température voisine de 20°C, versé sur 225 cm3 d'eau et lavé avec 100 cm3 d'oxyde de diéthyle. La phase organique est lavée avec 50 cm3 d'eau. Les phases aqueuses sont additionnées de 16,35 g d'hydrogénosulfate de tétrabutylammonium et extraites par 200 cm3 puis deux fois 100 cm3 de dichlorométhane. La phase organique est séchée sur sulfate de magnésium, filtrée et évaporée pour donner 22,6 g de (nitro-3 phényl)-2 éthylènesulfonate de tétrabutylammonium-(E), sous forme d'une huile utilisée telle quelle dans les synthèses ultérieures.

Le (nitro-3 phényl)-2 éthylènesulfonate d'éthyle-(E) peut être préparé selon la méthode décrite par J.C. CARRIETERO, M. DEMILLEQUAND, L. GHOSEZ, Tetrahedron, 43, (21), 5125-34 (1987).

### EXEMPLE 12

On opère comme à l'exemple 11, mais à partir de 3,2 g de (N,N-diéthylcarbamoylméthyl)-1 dihydro-2,3 isocyanato-3 oxo-2 phényl-5 1H-benzo[f]diazépine-1,4-(RS) et de 1,2 g d'(amino-3 benzyl)-5 tétrazole dans 50 cm3 de tétrahydrofuranne. Après traitement, on obtient 0,4 g de {{[(N,N-diéthyl-carbamoylméthyl)-1 dihydro-2,3 oxo-2 phényl-5 1H-benzo[f]diazépine-1,4 yl-3]-3 uréido}-3 benzyl}-5 tétrazole-(RS) fondant à 110°C.

L'(amino-3 benzyl)-5 tétrazole peut être obtenu selon la méthode décrite dans le brevet WO 91/13907.

### EXEMPLE 13

On opére comme à l'exemple 3, mais à partir de 1 g de {[(N,N-diéthylcarbamoylméthyl)-1 dihydro-2,3 oxo-2 phényl-5 1H-benzo[f]diazépine-1,4 yl-3]-3 uréido}-3 alpha-hydroxyimino phénylacétate de méthyle-(E)-(RS) et de 4,1 cm3 de soude 1N, dans un mélange de 10 cm3 d'éthanol et de 4 cm3 d'eau. Après traitement, on obtient 0,7 g d'acide {[(N,N-diéthylcarbamoylméthyl)-1 dihydro-2,3 oxo-2 phényl-5 1H-benzo[f]diazépine-1,4 yl-3]-3 uréido}-3 alpha-hydroxyimino phénylacétique-(E)-(RS), sous forme d'un solide fondant à 230°C.

Le {[(N,N-diéthyl-carbamoylméthyl)-1 dihydro-2,3 oxo-2 phényl-5 1H-benzo[f]diazépine-1,4 yl-3]-3 uréido}-3 alpha-hydroxyimino phénylacétate de méthyle-(E)-(RS) peut être obtenu de la manière suivante : On opère comme à l'exemple 12, mais à partir de 3,5 g de (N,N-diéthylcarbamoylméthyl)-1 dihydro-2,3 isocyanato-3 oxo-2 phényl-5 1H-benzo[f]diazépine-1,4-(RS) et de 1,5 g d'amino-3 alpha-hydroxyimino phénylacétate de méthyle-(E) dans 30 cm3 de tétrahydrofuranne. Après traitement, on obtient 2,9 g de {[(N,N-diéthyl-carbamoylméthyl)-1 dihydro-2,3 oxo-2 phényl-5 1H-benzo[f]diazépine-1,4 yl-3]-3 uréido}-3 alpha-hydroxyimino phénylacétate de méthyle-(E)-(RS), sous forme d'un solide utilisé tel quel dans les synthèses ultérieures.

L'amino-3 alpha-hydroxyimino phénylacétate de méthyle-(E) peut être obtenu de la manière suivante : On opère comme à l'exemple 9 pour la préparation de l'amino-3 alpha-tert-butoxycarbonylméthyloxyimino phénylacétate d'éthyle-(Z), mais à partir de 3,4 g de nitro-3 alpha-hydroxyimino phénylacétate de méthyle-(E) et de 0,034 g de dioxyde de platine dans 20 cm3 de méthanol. Après traitement, on obtient 2,9 g d'amino-3 alpha-hydroxyimino phénylacétate de méthyle-(E), sous forme d'un solide utilisé tel quel dans les synthèses ultérieures.

Le nitro-3 alpha-hydroxyimino phénylacétate de méthyle-(E) peut être obtenu comme à l'exemple 9 pour la préparation du nitro-3 alpha-hydroxyimino phénylacétate d'éthyle-(E), mais à partir de 15 g de nitro-3 phénylglyoxylate de méthyle et de 10 g de chlorhydrate d'hydroxylamine dans un mélange de 250 cm3 de pyridine et de 30 cm3 d'eau, pendant 30 minutes à 70°C. Après traitement, on obtient 3,4 g de nitro-3 alpha-hydroxyimino phénylacétate de méthyle-(E) fondant à 140°C et 6,1 g de nitro-3 alpha-hydroxyimino phénylacétate de méthyle-(Z) fondant à 190°C.

Le nitro-3 phénylglyoxylate de méthyle peut être préparé selon la méthode décrite par E. ADLEROVA, P. VEJDELKOVA, M. PROTIVA, Collection Czech. Chem. Commun., 29, 97-120 (1964).

### EXEMPLE 14

On opère comme à l'exemple 5, mais à partir de 0,6 g de {{[(N,N-diéthylcarbamoylméthyl)-1 dihydro-2,3 (fluoro-2 phényl)-5 oxo-2 1H-benzo[f]diazépine-1,4 yl-3]-3 uréido}-3 phénylthio}acétate de tert-butyle-(RS) et de 10 cm3 d'acide trifluoroacétique. Après traitement, on obtient 0,39 g d'acide {{[(N,N-diéthyl-carbamoylméthyl)-1 dihydro-2,3 (fluoro-2 phényl)-5 oxo-2 1H-benzo[f]diazépine-1,4 yl-3]-3 uréido}-3 phénylthio}acétique-(RS) fondant à 194°C.

Le {{[(N,N-diéthyl-carbamoylméthyl)-1 dihydro-2,3 (fluoro-2 phényl)-5 oxo-2 1H-benzo[f]diazépine-1,4 yl-3]-3 uréido}-3 phénylthio}acétate de tert-butyle-(RS) peut être obtenu comme à l'exemple 5, pour la préparation du {{[(N,N-diéthyl-carbamoylméthyl)-1 dihydro-2,3 oxo-2 phényl-5 1H-benzo[f]diazépine-1,4 yl-3]-3 uréido}-3 phénylthio}acétate de tert-butyle-(RS), mais en partant de 0,53 g d'(isocyanato-3 phénylthio)acétate de tert-butyle et de 0,7 g d'amino-3 (N,N-diéthyl-carbamoylméthyl)-1 dihydro-2,3 (fluoro-2 phényl)-5 oxo-2 1H-benzo[f]diazépine-1,4-(RS) dans 25 cm3 de tétrahydrofuranne. Après traitement, on obtient 0,6 g de {{[(N,N-diéthyl-carbamoylméthyl)-1 dihydro-2,3 (fluoro-2 phényl)-5 oxo-2 1H-benzo[f]diazépine-1,4 yl-3]-3 uréido}-3 phénylthio}acétate de tert-butyle-(RS), sous forme d'une meringue utilisée telle quelle dans les synthèses ultérieures.

L'amino-3 (N,N-diéthyl-carbamoylméthyl)-1 dihydro-2,3 (fluoro-2 phényl)-5 oxo-2 1H-benzo[f]diazépine-1,4-(RS) peut être obtenu comme à l'exemple 5, pour la préparation de l'amino-3 (N,N-diéthyl-carbamoylméthyl)-1 dihydro-2,3 oxo-2 phényl-5 1H-benzo[f]diazépine-1,4-(RS), mais en partant de 2,5 g de (N,N-diéthyl-carbamoylméthyl)-1 dihydro-2,3 (fluoro-2 phényl)-5 hydroxyiminino-3 oxo-2 1H-benzo[f]diazépine-1,4 et de 0,6 g de ruthénium à 5% sur charbon dans 60 cm3 de méthanol. Après traitement, on obtient 0,77 g d'amino-3 (N,N-diéthyl-carbamoylméthyl)-1 dihydro-2,3 (fluoro-2 phényl)-5 oxo-2 1H-benzo[f]diazépine-1,4-(RS), sous forme d'une meringue utilisée telle quelle dans les synthèses ultérieures.

La (N,N-diéthyl-carbamoylméthyl)-1 dihydro-2,3 (fluoro-2 phényl)-5 hydroxyiminino-3 oxo-2 1H-benzo[f]diazépine-1,4 peut être obtenue comme à l'exemple 5, pour la préparation de (N,N-diéthyl-carbamoylméthyl)-1 dihydro-2,3 hydroxyiminino-3 oxo-2 phényl-5 1H-benzo[f]diazépine-1,4, mais en partant de 5,7 g de (N,N-diéthyl-carbamoylméthyl)-1 dihydro-2,3 (fluoro-2 phényl)-5 oxo-2 1H-benzo[f]diazépine-1,4, de 3,47 g de tert-butylate de potassium et de 2,3 cm3 de nitrite d'isopentyle dans 200 cm3 de toluène. Après traitement, on obtient 5,1 g de (N,N-diéthyl-carbamoylméthyl)-1 dihydro-2,3 (fluoro-2 phényl)-5 hydroxyiminino-3 oxo-2 1H-benzo[f]diazépine-1,4 fondant à 190°C.

La (N,N-diéthyl-carbamoylméthyl)-1 dihydro-2,3 (fluoro-2 phényl)-5 oxo-2 1H-benzo[f]diazépine-1,4 peut être obtenue comme à l'exemple 5, pour la préparation de la (N,N-diéthyl-carbamoylméthyl)-1 dihydro-2,3 oxo-2 phényl-5 1H-benzo[f]diazépine-1,4, mais à partir de 5,08 g de dihydro-2,3 (fluoro-2 phényl)-5 oxo-2 1H-benzo[f]diazépine-1,4 de 3,43 g de N,N-diéthyl chloro-2 acétamide, de 0,33 g d'iodure de potassium et de 8,3 g de carbonate de potassium dans 60 cm3 de diméthylformamide sec. Après traitement, on obtient 5,8 g de (N,N-diéthyl-carbamoylméthyl)-1 dihydro-2,3 (fluoro-2 phényl)-5 oxo-2 1H-benzo[f]diazépine-1,4, sous forme d'une meringue utilisée telle quelle dans les synthèses ultérieures.

La dihydro-2,3 (fluoro-2 phényl)-5 oxo-2 1H-benzo[f]diazépine-1,4, peut être préparée selon la méthode décrite par L.H. STERNBACH, R.I. FRYER, W. METLESICS, E. REEDER; G. SACH, G. SAUCY et A. STEMPEL, J. Org. Chem., 27, 3788-3796 (1962).

### EXEMPLE 15

En opérant comme à l'exemple 1, mais à partir de 1,4 g de {{[dihydro-2,3 (diméthyl-3,3 pipéridino)carbonylméthyl-1 oxo-2 phényl-5 1H-benzo[f]diazépine-1,4 yl-3]-3 uréido}-4 phénylthio}acétate de tert-butyle-(RS) et de 5,6 cm3 d'acide trifluoroacétique, on obtient 0,59 g d'acide {{[dihydro-2,3 (diméthyl-3,3 pipéridino)carbonylméthyl-1 oxo-2 phényl-5 1H-benzo[f]diazépine-1,4 yl-3]-3 uréido}-4 phénylthio}acétique-(RS) fondant à 214°C.

Le {{[dihydro-2,3 (diméthyl-3,3 pipéridino)carbonylméthyl-1 oxo-2 phényl-5 1H-benzo[f]diazépine-1,4 yl-3]-3 uréido}-4 phénylthio}acétate de tert-butyle-(RS) peut être préparé d'une manière analogue à celle décrite à l'exemple 1 pour la préparation du {{[dihydro-2,3 oxo-2 phényl-5 (pyrrolidinyl-1)carbonylméthyl-1 1H-benzo[f]diazépine-1,4 yl-3]-3 uréido}-3 phénylthio}acétate de tert-butyle-(RS), mais à partir de 1,64 g d'amino-3 dihydro-2,3 (diméthyl-3,3 pipéridino)carbonylméthyl-1 oxo-2 phényl-5 1H-benzo[f]diazépine-1,4-(RS) et de 1,1 g d'(isocyanato-4 phénylthio)acétate de tert-butyle. On obtient ainsi 1,42 g de {{[dihydro-2,3 (diméthyl-3,3 pipéridino)carbonylméthyl-1 oxo-2 phényl-5 1H-benzo[f]diazépine-1,4 yl-3]-3 uréido}-4 phénylthio)acétate de tert-butyle-(RS) sous forme d'un solide utilisé tel quel dans les synthèses ultérieures.

L'(isocyanato-4 phénylthio)acétate de tert-butyle peut être préparé d'une manière analogue à celle décrite à l'exemple 1 pour la préparation de l'(isocyanato-3 phénylthio)acétate de tert-butyle, mais à partir de 3,1 g d'(amino-4 phénylthio)acétate de tert-butyle, de 1,6 cm3 de chloroformiate de trichlorométhyle et de 0,26 g de charbon. On obtient ainsi 2,3 g d'(isocyanato-4 phénylthio)acétate de tert-butyle sous forme d'une huile utilisée telle quelle dans les synthèses ultérieures.

L'(amino-4 phénylthio)acétate de tert-butyle peut être préparé d'une manière analogue à celle décrite à l'exemple 1 pour la préparation de l'(amino-3 phénylthio)acétate de tert-butyle, mais à partir de 5 g d'amino-4 thiophénol et de 7,8 cm3 de bromoacétate de tert-butyle. On obtient ainsi 7,45 g d'(amino-4 phénylthio)acétate de tert-butyle sous forme d'une huile utilisée telle quelle dans les synthèses ultérieures.

### EXEMPLE 16

En opérant comme à l'exemple 3, mais à partir de 0,8 g de {{[dihydro-2,3 (diméthyl-3,3 pipéridino)carbonylméthyl-1 oxo-2 phényl-5 1H-benzo[f]diazépine-1,4-yl-3]-3 uréido}-3 phényl}-3 propène-2 oate-(E) d'éthyle-(RS) et de 1,6 cm3 d'une solution aqueuse normale d'hydroxyde de sodium, on obtient 0,51 g d'acide {[dihydro-2,3 oxo-2 phényl-5 (diméthyl-3,3 pipéridino)carbonylméthyl-1 1H-benzo[f]diazépine-1,4-yl-3]-3 uréido}-3 phényl}-3 propène-2 oïque-(E) fondant à 216°C.

Le {{[dihydro-2,3 (diméthyl-3,3 pipéridino)carbonylméthyl-1 oxo-2 phényl-5 1H-benzo[f]diazépine-1,4 yl-3]-3 uréido}-3 phényl}-3 propène-2 oate-(E) d'éthyle-(RS) peut être préparé d'une manière analogue à celle décrite à l'exemple 3 pour la préparation du {{[(N-méthyl N-phényl-carbamoylméthyl)-1 oxo-2 phényl-5 dihydro-2,3 1H-benzo[f]diazépine-1,4 yl-3]-3 uréido}-3 phényl}-3 propène-2 oate-(E) d'éthyle-(RS), mais à partir de 1,7 g d'amino-3 dihydro-2,3 (diméthyl-3,3 pipéridino)carbonylméthyl-1 oxo-2 phényl-5 1H-benzo[f]diazépine-1,4-(RS) et de 0,9 g d'(isocyanato-3 phényl)-3 propène-2 oate-(E) d'éthyle. On obtient ainsi 0,85 g de {{[dihydro-2,3 (diméthyl-3,3 pipéridino)carbonylméthyl-1 oxo-2 phényl-5 1H-benzo[f]diazépine-1,4 yl-3]-3 uréido}-3 phényl}-3 propène-2 oate-(E) d'éthyle-(RS) sous forme d'un solide utilisé tel quel dans les synthèses ultérieures.

### EXEMPLE 17

En opérant comme à l'exemple 1, mais à partir de 2 g de {{[dihydro-2,3 oxo-2 phényl-5 (pyrrolidinyl-1)carbonylméthyl-1 1H-benzo[f]diazépine-1,4 yl-3]-3 uréido}-4 phénylthio}acétate de tert-butyle-(RS) et de 8 cm3 d'acide trifluoroacétique, on obtient 1,06 g d'acide {{[dihydro-2,3 oxo-2 phényl-5 (pyrrolidinyl-1)carbonylméthyl-1 1H-benzo[f]diazépine-1,4 yl-3]-3 uréido}-4 phénylthio}acétique-(RS) fondant à 190°C.

Le {{[dihydro-2,3 oxo-2 phényl-5 (pyrrolidinyl-1)carbonylméthyl-1 1H-benzo[f]diazépine-1,4 yl-3]-3 uréido}-4 phénylthio}acétate de tert-butyle-(RS) peut être préparé d'une manière analogue à celle décrite à l'exemple 1 pour la préparation du {{[dihydro-2,3 oxo-2 phényl-5 (pyrrolidinyl-1)carbonylméthyl-1 1H-benzo[f]diazépine-1,4 yl-3]-3 uréido}-3 phénylthio}acétate de tert-butyle-(RS), mais à partir de 6,3 g d'amino-3 dihydro-2,3 oxo-2 phényl-5 (pyrrolidinyl-1)carbonylméthyl-1 1H-benzo[f]diazépine-1,4-(RS) et de 5,1 g d'(isocyanato-4 phénylthio)acétate de tert-butyle. On obtient ainsi 2 g de {{[dihydro-2,3 oxo-2 phényl-5 (diméthyl-3,3 pipéridino)carbonylméthyl-1 1H-benzo[f]diazépine-1,4 yl-3]-3 uréido)-4 phénylthio)acétate de tert-butyle-(RS) sous forme d'un solide utilisé tel quel dans les synthèses ultérieures.

### EXEMPLE 18

En opérant comme à l'exemple 10, mais à partir de 2 g d'isocyanato-3 dihydro-2,3 méthyl-1 oxo-2 phényl-5 1H-benzo[f]diazépine-1,4-(RS) et de 2,9 g d'(amino-3 phényl)-1 éthanesulfonate-(RS) de tétrabutylammonium, on obtient 1,4 g d'un mélange des isomères de {[(dihydro-2,3 oxo-2 phényl-5 méthyl-1 1H-benzo[f]diazépine-1,4 yl-3-(RS))-3 uréido]-3 phényl}-1 éthanesulfonate de potassium-(RS) fondant à 258°C.

L'(amino-3 phényl)-1 éthanesulfonate de tétrabutylammonium-(RS) peut être préparé comme décrit à l'exemple 2 pour la préparation de l'amino-3 phénylméthanesulfonate de tétrabutylammonium, mais à partir de 21 g de (nitro-3 phényl)-1 éthanesulfonate de tetrabutylammonium-(RS) et de 0,9 g de palladium à 5% sur charbon dans 300 cm3 d'éthanol. Après traitement, on obtient 19 g d'(amino-3 phényl)-1 éthanesulfonate de tetrabutylammonium-(RS) sous forme d'une huile utilisée telle quelle dans les synthèses ultérieures.

Le (nitro-3 phényl)-1 éthanesulfonate de tétrabutylammonium-(RS) peut être préparé de la manière suivante : un mélange de 10,4 g de (bromo-1 éthyl)-3 nitrobenzène-(RS) et de 8,5 g d'hydrogénosulfite de sodium dans 109 cm³ d'eau est agité à une température voisine de 70°C pendant deux heures. Après refroidissement à une température voisine de 20°C, la solution est versée dans un litre d'une solution aqueuse de 89 g d'hydrogénosulfate de tetrabutylammonium et extraite par trois fois 100 cm3 de dichlorométhane. Les extraits sont réunis, lavés par 50 cm3 d'eau, séchés sur sulfate de magnésium et concentrés sous pression réduite. On obtient ainsi 21 g de (nitro-3 phényl)-1 éthanesulfonate de tétrabutylammonium-(RS) sous forme d'une huile utilisée telle quelle dans les synthèses ultérieures.

Le (bromo-1 éthyl)-3 nitrobenzène-(RS) peut être préparé selon la méthode décrite par T.Y. SHEN, N.P. JENSEN, D.H. MINSKER, brevet DE 2331292.

Le dihydro-2,3 isocyanato-3 méthyl-1 oxo-2 phényl-5 1H-benzo[f]diazépine-1,4-(RS) peut être préparé d'une manière analogue à celle décrite à l'exemple 10 pour la préparation du dihydro-2,3 isocyanato-3 oxo-2 phényl-5 (pyrrolidinyl-1)carbonylméthyl-1 1H-benzo[f]diazépine-1,4-(RS), mais à partir de 2 g d'amino-3 dihydro-2,3 méthyl-1 oxo-2 phényl-5 1H-benzo[f]diazépine-1,4-(RS), de 0,09 g de charbon et de 0,91 cm3 de chloroformiate de trichlorométhyle. On obtient ainsi 2,13 g de dihydro-2,3 isocyanato-3 méthyl-1 oxo-2 phényl-5 1H-benzo[f]diazépine-1,4-(RS) sous forme d'une huile utilisée telle quelle dans les synthèses ultérieures.

### EXEMPLE 19

En opérant comme à l'exemple 1, mais à partir de 1 g de (+)-{{[dihydro-2,3 oxo-2 phényl-5 (pyrrolidinyl-1)carbonylméthyl-1 1H-benzo[f]diazépine-1,4 yl-3]-3 uréido}-3 phénylthio}acétate de tert-butyle et de 4 cm3 d'acide trifluoroacétique, on obtient 0.6 g d'acide (+)-{{[dihydro-2,3 oxo-2 phényl-5 (pyrrolidinyl-1)carbonylméthyl-1 1H-benzo[f]diazépine-1,4 yl-3]-3 uréido}-3 phénylthio}acétique dont le pouvoir rotatoire est [α]²⁰_{D}= +33,7° (c = 0.994; méthanol).

Le (+)-{{[dihydro-2,3 oxo-2 phényl-5 (pyrrolidinyl-1)carbonylméthyl-1 1H-benzo[f]diazépine-1,4 yl-3]-3 uréido}-3 phénylthio}acétate de tert-butyle peut être obtenu de la manière suivante : 2 g de {{[dihydro-2,3 oxo-2 phényl-5 (pyrrolidinyl-1)carbonylméthyl-1 1H-benzo[f]diazépine-1,4 yl-3]-3 uréido}-3 phénylthio}acétate de tert-butyle-(RS) sont séparés par chromatographie liquide à haute performance sur 400 g de support constitué de silice enrobée de tris-(diméthyl-3,5 phényl)carbamate de cellulose préparé selon J. Amer. Chem, Soc., 106, 5357 (1984) et contenu dans une colonne de 23 cm de longueur et de 6 cm de diamètre avec commme phase mobile de l'éthanol au débit de 35 cm3/minute en éluant successivement :
- 1 g de (+)-{{[dihydro-2,3 oxo-2 phényl-5 (pyrrolidinyl-1)carbonylméthyl-1 1H-benzo[f]diazépine-1,4 yl-3]-3 uréido}-3 phénylthio)acétate de tert-butyle dont le pouvoir rotatoire est [α]²⁰_{D} = +32,6° (c = 0.994; méthanol), puis
- 0,840 g d'un mélange qui est recyclé dans les mêmes conditions pour donner 0,7 g de (-)-{{[dihydro-2,3 oxo-2 phényl-5 (pyrrolidinyl-1)carbonylméthyl-1 1H-benzo[f]diazépine-1,4 yl-3]-3 uréido}-3 phénylthio}acétate de tert-butyle dont le pouvoir rotatoire est [α]²⁰_{D} = -32,6° (c = 0,614; méthanol).

### EXEMPLE 20

En opérant comme à l'exemple 1, mais à partir de 0,7 g de (-)-{{[dihydro-2,3 oxo-2 phényl-5 (pyrrolidinyl-1)carbonylméthyl-1 1H-benzo[f]diazépine-1,4 yl-3]-3 uréido}-3 phénylthio}acétate de tert-butyle et de 3 cm3 d'acide trifluoroacétique, on obtient 0.4 g d'acide (-)-{{[dihydro-2,3 oxo-2 phényl-5 (pyrrolidinyl-1)carbonylméthyl-1 1H-benzo[f]diazépine-1,4 yl-3]-3 uréido}-3 phénylthio}acétique dont le pouvoir rotatoire est [α]²⁰_{D}= -33,7° (c = 0.961; méthanol).

### EXEMPLE 21

En opérant comme à l'exemple 1, mais à partir de 0,4 g d'un mélange des isomères du {{[dihydro-2,3 oxo-2 phényl-5 (pyrrolidinyl-1)carbonylméthyl-1 1H-benzo[f]diazépine-1,4 yl-3-(RS)]-3 uréido}-3 phénylsulfinyl}acétate de tert-butyle et de 3 cm3 d'acide trifluoroacétique, on obtient, après recristallisation dans l'isopropanol, 0,138 g d'un mélange des isomères de l'acide {{[dihydro-2,3 oxo-2 phényl-5 (pyrrolidinyl-1)carbonylméthyl-1 1H-benzo[f]diazépine-1,4 yl-3-(RS)]-3 uréido}-3 phénylsulfinyl}acétique-(RS) fondant à 180°C.

Le mélange des isomères du {{[dihydro-2,3 oxo-2 phényl-5 (pyrrolidinyl-1)carbonylméthyl-1 1H-benzo[f]diazépine-1,4 yl-3-(RS)]-3 uréido}-3 phénylsulfinyl}acétate de tert-butyle-(RS) peut être obtenu de la manière suivante : à une solution de 2 g de {{[dihydro-2,3 oxo-2 phényl-5 (pyrrolidinyl-1)carbonylméthyl-1 1H-benzo[f]diazépine-1,4 yl-3]-3 uréido}-3 phénylthio}acétate de tert-butyle-(RS) dans 16 cm3 de méthanol, on ajoute une solution de 3,7 g d'oxone^{R} dans 22 cm3 d'eau. La suspension est agitée pendant 16 heures à une température voisine de 20°C, filtrée et le solide lavé par deux fois 10 cm3 d'eau puis deux fois 10 cm3 d'oxyde de diéthyle. Par chromatographie sur silice (éluant acétate d'éthyle), on élue successivement :
- 1,05 g de {{[dihydro-2,3 oxo-2 phényl-5 (pyrrolidinyl-1)carbonylméthyl-1 1H-benzo[f]diazépine-1,4 yl-3]-3 uréido}-3 phénylsulfonyl}acétate de tert-butyle-(RS) sous forme d'un solide utilisé tel quel dans les synthèses ultérieures, puis
- 0,4 g d'un mélange des isomères du {{[dihydro-2,3 oxo-2 phényl-5 (pyrrolidinyl-1)carbonylméthyl-1 1H-benzo[f]diazépine-1,4 yl-3-(RS)]-3 uréido}-3 phénylsulfinyl}acétate de tert-butyle-(RS) sous forme d'un solide utilisé tel quel dans les synthèses ultérieures.

### EXEMPLE 22

En opérant comme à l'exemple 1, mais à partir de 1 g de {{[dihydro-2,3 oxo-2 phényl-5 (pyrrolidinyl-1)carbonylméthyl-1 1H-benzo[f]diazépine-1,4 yl-3]-3 uréido}-3 phénylsulfonyl}acétate de tert-butyle-(RS) et de 8 cm3 d'acide trifluoroacétique, on obtient 0,61 g d'acide {{[dihydro-2,3 oxo-2 phényl-5 (pyrrolidinyl-1)carbonylméthyl-1 1H-benzo[f]diazépine-1,4 yl-3]-3 uréido}-3 phénylsulfonyl}acétique-(RS) fondant à 210°C.

### EXEMPLE 23

A une suspension de 1,4 g d'un mélange des isomères du {{[dihydro-2,3 oxo-2 phényl-5 (pyrrolidinyl-1)carbonylméthyl-1 1H-benzo[f]diazépine-1,4 yl-3]-3 uréido}-3 phénylthio}-2 propionate d'éthyle dans 140 cm3 d'éthanol, on ajoute, à une température voisine de 20°C, une solution de 0,091 g d'hydrate de lithine dans 11 cm3 d'eau. Le mélange réactionnel est agité pendant 72 heures à une température voisine de 20°C, puis concentré sous pression réduite. Le résidu est dilué par 50 cm3 d'eau et amené à pH 1 par une solution aqueuse 1N d'acide chlorhydrique. Le solide qui précipite est filtré, lavé par deux fois 10 cm3 d'eau puis deux fois 10 cm3 d'oxyde de diéthyle. Après recristallisation dans le méthanol et battage dans l'acétonitrile, on obtient 0,64 g d'un mélange des isomères de l'acide {{[dihydro-2,3 oxo-2 phényl-5 (pyrrolidinyl-1)carbonylméthyl-1 1H-benzo[f]diazépine-1,4 yl-3-(RS)]-3 uréido}-3 phénylthio}-2 propionique-(RS) fondant à 184°C.

Le mélange des isomères du {{[dihydro-2,3 oxo-2 phényl-5 (pyrrolidinyl-1)carbonylméthyl-1 1H-benzo[f]diazépine-1,4 yl-3]-3 uréido}-3 phénylthio}-2 propionate d'éthyle peut être obtenu d'une manière analogue à celle décrite à l'exemple 1 pour la préparation du {{[dihydro-2,3 oxo-2 phényl-5 (pyrrolidinyl-1)carbonylméthyl-1 1H-benzo[f]diazépine-1,4 yl-3]-3 uréido}-3 phénylthio}acétate de tert-butyle-(RS), mais à partir de 1,6 g d'amino-3 dihydro-2,3 oxo-2 phényl-5 (pyrrolidinyl-1)carbonylméthyl-1 1H-benzo[f]diazépine-1,4-(RS) et de 1,1 g d'(isocyanato-3 phénylthio)-2 propionate d'éthyle. On obtient ainsi 1,7 g de mélange des isomères du {{[dihydro-2,3 oxo-2 phényl-5 (pyrrolidinyl-1)carbonylméthyl-1 1H-benzo[f]diazépine-1,4 yl-3]-3 uréido}-3 phénylthio}-2 propionate d'éthyle sous forme d'un solide utilisé tel quel dans les synthèses ultérieures.

L'(isocyanato-3 phénylthio)-2 propionate d'éthyle peut être obtenu d'une manière analogue à celle décrite à l'exemple 1 pour la préparation de l'(isocyanato-3 phénylthio)acétate de tert-butyle, mais à partir de 1 g d'(amino-3 phénylthio)-2 propionate d'éthyle, de 0,54 cm³ de chloroformiate de trichlorométhyle et de 0,09 g de charbon. On obtient ainsi 1,18 g d'(isocyanato-3 phénylthio)-2 propionate d'éthyle sous forme d'une huile utilisée telle quelle dans les synthèses ultérieures.

L'(amino-3 phénylthio)-2 propionate d'éthyle peut être préparé d'une manière analogue à celle décrite à l'exemple 1 pour la préparation de l'(amino-3 phénylthio)acétate de tert-butyle, mais à partir de 9,25 g d'amino-3 thiophénol et de 18,5 cm3 de trifluorométhylsulfonyloxy-2 propionate d'éthyle et de 10,2 g de carbonate de potassium dans 200 cm3 d'acétonitrile. On obtient ainsi 4,04 g d'(amino-3 phénylthio)-2 propionate d'éthyle sous forme d'une huile utilisée telle quelle dans les synthèses ultérieures.

Le trifluorométhylsulfonyloxy-2 propionate d'éthyle peut être préparé selon la méthode décrite par U. BURKARD, F. EFFENBERGER Chem. Ber., 119, 1594 (1986).

### EXEMPLE 24

2,74 g de {{[N,N-diéthyl-carbamoylméthyl)-1 dihydro-2,3 oxo-2 phényl-5 1H-benzo[f]diazépine-1,4 yl-3]-3 uréido}-3 phénylthio}acétate de tert-butyle-(RS) sont dissous lentement dans 12 cm³ d'acide trifluoroacétique. Après 1 heure d'agitation à une température voisine de 20°C, on ajoute 50 cm³ de méthanol et agite 18 heures à une température voisine de 20°C. On obtient une suspension qui est filtrée. Le filtrat est évaporé à sec sous pression réduite (2,7 kPa) pour donner une huile qui est chromatographiée sur une colonne de 4 cm de diamètre contenant 600 cm³ de silice, en éluant d'abord avec 500 cm³ de dichlorométhane puis avec le mélange dichlorométhane-méthanol (93/3 en volumes), et en recueillant des fractions de 125 cm³. Les fractions comprises entre 2625 et 3125 cm³ sont réunies et évaporées à sec pour donner 1,4 g de solide. Par recristallisation dans 50 cm³ de méthanol aqueux (80/20 en volumes), on obtient 0,93 g de {{[N,N-diéthyl-carbamoylméthyl)-1 dihydro-2,3 oxo-2 phényl-5 1H-benzo[f]diazépine-1,4 yl-3]-3 uréido}-3 phénylthio}acétate de tert-butyle-(RS), sous forme d'un solide blanc fondant à 198°C.

### Exemple 25

On opère comme à l'exemple 11, mais à partir de 2 g de (N,N-diéthylaminocarbonylméthyl)-1 dihydro-2,3 isocyanato-3 oxo-2 phényl-5 1H-benzo[f]diazépine-1,4-(RS) et de 0,92 g d'acide N-{[(amino-3 phényl)méthyl)-acétohydroxamique dans 40 cm³ de tétrahydrofuranne. Après traitement, on obtient 0,24 g d'acide N-{{{[(N,N-diéthylaminocarbonylméthyl)-1 dihydro-2,3 oxo-2 phényl-5 1H-benzo[f]diazépine-1,4 yl-3]-3 uréido}-3 phényl}méthyl}-acétohydroxamique-(RS), sous forme d'un solide blanc fondant à 186°C.

L'acide N-{amino-3 phényl)méthyl}-acétohydroxamique peut être préparé comme décrit dans l'exemple 2 pour la préparation de l'amino-3 phénylsulfonate de tétrabutylammonium, mais à partir de 1,92 g d'acide N-{(nitro-3 phényl)méthyl}acétohydroxamique et de 0,2 g de palladium à 10 % sur charbon dans 100 cm³ de méthanol. Après traitement, on obtient 1,73 g d'acide N-{amino-3 phényl)méthyl}-acétohydroxamique sous forme d'une huile utilisée telle quelle dans les synthèses ultérieures.

L'acide N-{(nitro-3 phényl)méthyl}-acétohydroxamique peut être obtenu de la manière suivante : à une solution de 3,55 g de N-acétoxy N-{(nitro-3 phényl)méthyl}-acétamide dans 120 cm³ de méthanol on ajoute, à une température voisine de 25°C, 22 cm³ d'une solution aqueuse 17N d'ammoniaque. Le milieu réactionnel est agité pendant 2 heures à une température voisine de 25°C, puis concentré sous pression réduite. Le résidu est dissous dans 100 cm³ d'un solution aqueuse saturée en chlorure de sodium, séchée sur sulfate de magnésium et concentrée sous pression réduite. On obtient ainsi 2,46 g d'acide N-{(nitro-3 phényl)méthyl}-acétohydroxamique fondant à 80°C.

Le N-acétoxy N-{(nitro-3 phényl)méthyl}-acétamide peut être obtenu de la manière suivante : une solution de 3,35 g de N-{(nitro-3 phényl)méthylhydroxylamine dans 8 cm³ d'anhydride acétique et 80 cm³ d'acide acétique est chauffée au reflux prendant 3 heures. Après refroidissement à une température voisine de 25°C, le milieu réactionnel est concentré sous pression réduite. Le résidu est dissous dans 100 cm³ d'oxyde de diéthyle et la phase organique est lavée par 50 cm³ d'une solution aqueuse saturée en chlorure de sodium, séchée sur sulfate de magnésium et concentrée sous pression réduite. On obtient ainsi 3,55 g de N-acétoxy N-{(nitro-3 phényl)méthyl}-acétamide sous forme d'une huile utilisée telle quelle dans les synthèses ultérieures.

La N-{(nitro-3 phényl)méthyl}-hydroxylamine peut être obtenue de la manière suivante : à une solution de 5 g de nitro-3 benzaldoxime dans 120 cm³ d'acide acétique, on ajoute, par portions, en deux heures, à une température voisine de 25°C, 3,78 g de cyanoborohydrure de sodium. Le milieu réactionnel est agité pendant 16 heures à une température voisine de 25°C, puis versé dans un mélange de 500 cm³ d'acétate d'éthyle et de 150 cm³ d'une solution aqueuse à 30 % de potasse. La phase organique est séparée par décantation et la phase aqueuse est extraite par trois fois 500 cm³ d'acétate d'éthyle. Les phases organiques sont réunies, séxhées sur sulfate de magnésium et concentrées sous pression réduite. Le résidu est purifié par chromatographie sur 200 cm³ de silice [éluant duchlorométhane-méthanol (98/2 en volumes)]. Les fractions contenant le produit attendu sont réunies et concentrées sous pression réduite. On obtient ainsi 3,43 g de N-{(nitro-3 phényl)méthyl}-hydroxylamine sous forme d'une huile utilisée telle quelle dans les synthèses ultérieures.

Les médicaments selon l'invention sont constitués par un compose de formule (I) sous forme libre ou sous forme d'un sel, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles dans le traitement et la prévention des désordres liés à la CCK et à la gastrine au niveau du système nerveux et de l'appareil gastrointestinal. Ces composés peuvent donc être utilisés dans le traitement et la prévention des psychoses, des attaques de panique, des troubles anxieux, de la maladie de Parkinson, de la diskynésie tardive, du syndrome du colon irritable, de la pancréatite aiguë, des ulcères, des désordres de la motilité intestinale, de certaines tumeurs sensibles à la CCK, des troubles de la mémoire, comme analgésiques, comme potentialisateur de l'activité analgésique des médicaments analgésiques narcotiques et non narcotiques, dans le sevrage aux traitements chroniques ou abus de médicaments et d'alcool, comme régulateur de l'appétit et pour controler la constriction de la pupille de l'oeil.

Les doses dépendent de l'effet recherché, de la durée du traitement et de la voie d'administration utilisée; elles sont généralement comprises entre 0,05 g et 1 g par jour par voie orale pour un adulte avec des doses unitaires allant de 10 mg à 500 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :

| | |
|---|---|
| - Acide {{[dihydro-2,3 oxo-2 phényl-5 (pyrrolidinyl-1) carbonylméthyl-1 1H-benzo[f]diazépine-1,4 yl-3]-3 uréido}-3 phénylthio}acétique-(RS) | 50 mg |
| - Cellulose | 18 mg |
| - Lactose | 55 mg |
| - Silice colloïdale | 1 mg |
| - Carboxyméthylamidon sodique | 10 mg |
| - Talc | 10 mg |
| - Stéarate de magnésium | 1 mg |

### EXEMPLE B

On prépare selon la technique habituelle de comprimés dosés à 50 mg de produit actif ayant la composition suivante :

| | |
|---|---|
| - acide {{[dihydro-2,3 oxo-2 phényl-5 (diméthyl-3,3 pipéridino) carbonylméthyl-1 1H-benzo[f] diazépine-1,4 yl-3]-3 uréido}-3 phénylthio}acétique-(RS) | 50 mg |
| - Lactose | 104 mg |
| - Cellulose | 40 mg |
| - Polyvidone | 10 mg |
| - Carboxyméthylamidon sodique | 22 mg |
| - Talc | 10 mg |
| - Stéarate de magnésium | 2 mg |
| - Silice colloïdale | 2 mg |
| - Mélange d'hydroxyméthylcellulose, glycérine, oxyde de titane (72-3,5-24,5) q.s.p. 1 comprimé pelliculé terminé à | 245 mg |

### EXEMPLE C

On prépare une solution injectable contenant 10 mg de produit actif ayant la composition suivante :

| | |
|---|---|
| - Acide {[(N-méthyl N-phényl-carbamoylméthyl)-1 oxo-2 phényl-5 dihydro-2,3 1H-benzo[f]diazépine-1,4 yl-3]-3 uréido}-3 phényl-3 propène-2 oïque-(E)-(RS) | 10 mg |
| - Acide benzoïque | 80 mg |
| - Alcool benzylique | 0,06 cm³ |
| - Benzoate de sodium | 80 mg |
| - Ethanol à 95 % | 0,4 cm³ |
| - Hydroxyde de sodium | 24 mg |
| - Propylène glycol | 1,6 cm³ |
| - Eau q.s.p. | 4 cm3 |

## Revendications

1. Composés de formule : 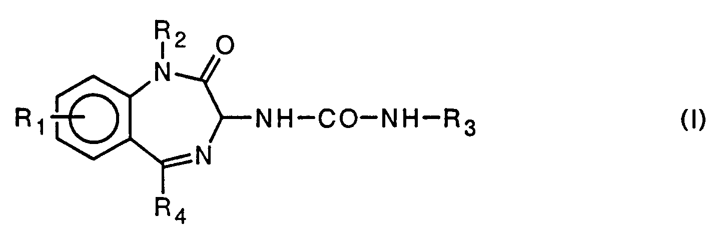 dans laquelle
- R₁ représente un atome d'hydrogène ou d'halogène ou un radical alkyle, alcoxy, alkylthio, nitro, hydroxy ou cyano,
- R₂ représente un radical alkyle ou une chaîne -CH(R₅)-CO-R₆ dans laquelle R₅ représente un atome d'hydrogène ou un radical alkyle, alcoxycarbonyle ou phényle éventuellement substitué (par un ou plusieurs substituants choisis parmi les atomes d'halogène et les radicaux alkyle, alcoxy, alkylthio et nitro) et R₆ représente un radical alcoxy, cycloalkyloxy éventuellement substitué (par au moins un radical alkyle), cycloalkylalkyloxy, phénylalkyloxy, polyfluoroalkyloxy, cinnamyloxy, un reste -NR₇R₈ dans lequel R₇ et R₈ identiques ou différents représentent un atome d'hydrogène ou un radical alkyle, phényle éventuellement substitué (par un ou plusieurs substituants choisis parmi les atomes d'halogène et les radicaux alkyle, alcoxy et alkylthio), cycloalkylalkyle, cycloalkyle, indanyle, phénylalkyle ou bien R₇ et R₈ forment ensemble avec l'atome d'cote auquel ils sont rattachés un hétérocycle mono ou polycyclique saturé ou insaturé contenant 4 à 9 atomes de carbone et un ou plusieurs hétéroatomes (O, S, N) et éventuellement substitué par un ou plusieurs radicaux alkyle, alcoxy, alcoxycarbonyle, dialkylcarbamoyle, phényle ou par un cycle spiromonocyclique formé par combinaison d'un atome de l'hétérocycle avec 4 ou 5 autres atomes de carbone dont l'un ou plusieurs de ces derniers peuvent éventuellement être remplacés par des hétéroatomes (O, S, N),
- R₃ représente (a) un radical phényle substitué par un ou plusieurs substituants choisis parmi les radicaux -alk-SO₃H, -alk-PO₃H₂, -CH=NOH, -CH=NO-alk-COOX, -S-alk-COOX, -SO-alk-COOX, -SO₂-alk-COOX, -CH=CH-COOX, -alk-CO-NHOH, -C(=NOH)-COOX, -alk-N(OH)-CO-alk, -alk-SO₂H, -CH=CH-SO₃H, -C(COOX)=N-O-alk-COOX, tétrazolylalkyle ou un groupe de formule : 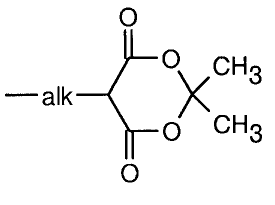 ou (b) un cycle de formule : 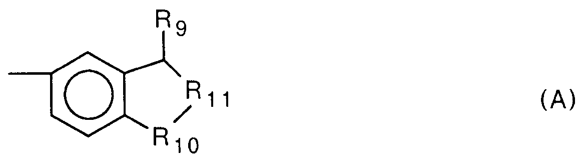 dans laquelle R₉ représente un radical =NOX, =NO-alk-COOX, =CH-COOX, -alk-COOX, -alk-SO₂H ou -alk-SO₃H, R₁₀ représente un atome d'oxygène ou de soufre ou un radical méthylène ou alkylimino et R₁₁ représente un radical méthylène ou éthylène,
- R₄ représente un radical pyridyle ou phényle éventuellement substitué par un ou plusieurs substituants choisis parmi les atomes d'halogène et les radicaux alkyle, alcoxy, hydroxy, carboxy et nitro,
- alk représente un radical alkyle ou alkylène,
- X représente un atome d'hydrogène ou un radical alkyle,
à l'exception des composés pour lesquels R₁ représente un atome d'hydrogène ou 1 ou 2 halogène ou méthyle, R₂ représente un radical alkyle ou -CH(R₅)-CO-R₆ dans lequel R₅ représente un atome d'hydrogène et R₆ représente un reste pyrrolidinyl-1, R₃ représente un radical phényle substitué en position 3 par un radical tétrazolyl-5 méthyle et R₄ représente un radical phényle éventuellement substitué par un ou deux atomes d'halogène ou méthyle,
étant entendu que les radicaux alkyle, alkylène et alcoxy et les portions alkyle, alkylène et alcoxy contiennent 1 à 4 atomes de carbone en chaîne droite ou ramifiée, et les radicaux et portions cycloalkyle contiennent 3 à 12 atomes de carbone,
ainsi que leurs sels et leurs racémiques et énantiomères lorsqu'ils comportent au moins un centre asymétrique.

2. Composés de formule (I) selon la revendication 1 pour lesquels R₇ et R₈ forment avec l'atome d'azote auquel ils sont rattachés un hétérocycle choisi parmi les cycles pipéridino, perhydroazépinyl-1, tétrahydro-1,2,3,6 pyridyl-1, tétrahydro-1,2,3,4 quinolyl-1, pyrrolidinyl-1, tétrahydro-1,2,3,4 isoquinolyl-2, morpholino, thiomorpholino ou indolyl-1, ces cycles pouvant être éventuellement substitués par un ou plusieurs radicaux alkyle, leurs sels et leurs racémiques et énantiomères.

3. Composés de formule (I) selon la revendication 1 pour lesquels R₁ représente un atome d'hydrogène, R₂ représente un radical alkyle ou -CH(R₅)COR₆ dans lequel R₅ représente un atome d'hydrogène et R₆ représente un radical -NR₇R₈ dans lequel R₇ et R₈ forment ensemble avec l'atome d'azote auquel ils sont rattachés un hétérocycle, R₃ représente (a) un radical phényle substitué par un ou plusieurs substituants choisis parmi les radicaux -alk-SO₃H, -alk-PO₃H₂, -CH=NOH, -CH=NO-alk-COOX, -S-alk-COOX, -SO-alk-COOX, -SO₂-alk-COOX, -CH=CH-COOX, -alk-CO-NHOH, -C(=NOH)-COOX, -alk-N(OH)-CO-alk, -alk-SO₂H, -CH=CH-SO₃H, -C(COOX)=N-O-alk-COOX, tétrazolylalkyle ou un groupe de formule : 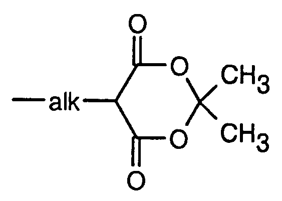 ou (b) un cycle de formule : 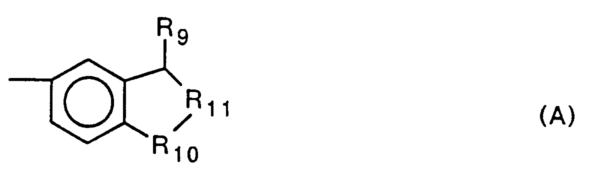 dans laquelle R₉ représente un radical =NOX, =NO-alk-COOX, =CH-COOX, -alk-COOX, -alk-SO₂H ou -alk-SO₃H, R₁₀ représente un atome d'oxygène ou de soufre ou un radical méthylène ou alkylimino et R₁₁ représente un radical méthylène ou éthylène et R₄ représente un radical phényle.

4. Composés de formule (I) selon la revendication 3 pour lesquels -NR₇R₈ représente un cycle pyrrolidinyl-1 ou pipéridino, ces cycles étant non substitués ou substitués par un ou plusieurs radicaux alkyle, leurs sels, leurs racémiques et énantiomères

5. Les composés suivants :
- acide {{[dihydro-2,3 oxo-2 phényl-5 (pyrrolidinyl-1)carbonylméthyl-1 1H-benzo[f]diazépine-1,4 yl-3]-3 uréido}-3 phénylthio}acétique-(RS),
- acide [(dihydro-2,3 méthyl-1 oxo-2 phényl-5 1H-benzo[f]diazépine-1,4 yl-3)-3 uréido]-3 phénylméthanesulfonique-(RS),
- acide {[(N-méthyl N-phényl-carbamoylméthyl)-1 oxo-2 phényl-5 dihydro-2,3 1H-benzo[f]diazépine-1,4 yl-3]-3 uréido}-3 phényl-3 propène-2(E)oïque-(RS),
- acide {{[dihydro-2,3 oxo-2 phényl-5 (diméthyl-3,3 pipéridino)carbonylméthyl-1 1H-benzo[f] diazépine-1,4 yl-3]-3 uréido}-3 phénylthio}acétique-(RS),
- acide {{[(N,N-diéthyl-carbamoylméthyl)-1 dihydro-2,3 oxo-2 phényl-5 1H-benzo[f]diazépine-1,4 yl-3]-3 uréido}-3 phénylthio}acétique-(RS),
- N-(dihydro-2,3 méthyl-1 oxo-2 phényl-5 1H-benzo[f]diazépine-1,4 yl-3) N'-(hydroxyimino-1 indanyl-6) urée-(E)-(RS),
- acide {dihydro-3,4 [(dihydro-2,3 méthyl-1 oxo-2 phényl-5 1H-benzo[f]diazépine-1,4 yl-3)-3 uréido]-6 2H-benzopyrannylidène-4}-2 acétique-(E)-(RS),
- acide {[dihydro-2,3 oxo-2 phényl-5 (pyrrolidinyl-1)carbonylméthyl-1 1H-benzo[f]diazépine-1,4 yl-3]-3 uréido}-3 phénylméthylènaminooxy-2 acétique-(E)-(RS),
- acide {{[(dihydro-2,3 oxo-2 phényl-5 (pyrrolidinyl-1)carbonylméthyl-1 1H-benzo[f]diazépine-1,4 yl-3]-3 uréido}-3 phényl}-2 (carboxyméthyloxyimino)-2 acétique-(RS),
- {{[dihydro-2,3 oxo-2 phényl-5 (pyrrolidinyl-1)carbonylméthyl-1 1H-benzo[f]diazépine-1,4 yl-3]-3 uréido}-3 benzyl}-5 diméthyl-2,2 dioxanne-1,3 dione-4,6-(RS),
- acide {{[(N,N-diéthyl-carbamoylméthyl)-1 dihydro-2,3 oxo-2 phényl-5 1H-benzo[f]diazépine-1,4 yl-3]-3 uréido}-3 phényl}-2 éthylènesulfonique-(E)-(RS),
- {{[(N,N-diéthyl-carbamoylméthyl)-1 dihydro-2,3 oxo-2 phényl-5 1H-benzo[f]diazépine-1,4 yl-3]-3 uréido}-3 benzyl}-5 tétrazole-(RS),
- acide {{(N,N-diéthyl-carbamoylméthyl)-1 dihydro-2,3 oxo-2 phényl-5 1H-benzo[f]diazépine-1,4 yl-3]-3 uréido}-3 alpha-hydroxyimino phénylacétique-(E)-(RS),
- acide {{[(N,N-diéthyl-carbamoylméthyl)-1 dihydro-2,3 (fluoro-2 phényl)-5 oxo-2 1H-benzo[f]diazépine-1,4 yl-3]-3 uréido}-3 phénylthio}acétique-(RS),
- acide {{[dihydro-2,3 (diméthyl-3,3 pipéridino)carbonylméthyl-1 oxo-2 phényl-5 1H-benzo[f]diazépine-1,4 yl-3]-3 uréido}-4 phénylthio}acétique-(RS),
- acide {[dihydro-2,3 oxo-2 phényl-5 (diméthyl-3,3 pipéridino)carbonylméthyl-1 1H-benzo[f]diazépine-1,4-yl-3]-3 uréido}-3 phényl}-3 propène-2 oïque-(E)-(RS),
- acide {{[dihydro-2,3 oxo-2 phényl-5 (pyrrolidinyl-1)carbonylméthyl-1 1H-benzo[f]diazépine-1,4 yl-3]-3 uréido}-4 phénylthio}acétique-(RS),
- {[(dihydro-2,3 oxo-2 phényl-5 méthyl-1 1H-benzo[f]diazépine-1,4 yl-3-(RS))-3 uréido]-3 phényl}-1 éthanesulfonate de potassium-(RS),
- acide (+)-{{[dihydro-2,3 oxo-2 phényl-5 (pyrrolidinyl-1)carbonylméthyl-1 1H-benzo[f]diazépine-1,4 yl-3]-3 uréido}-3 phénylthio}acétique,
- acide (-)-{{[dihydro-2,3 oxo-2 phényl-5 (pyrrolidinyl-1)carbonylméthyl-1 1H-benzo[f]diazépine-1,4 yl-3]-3 uréido}-3 phénylthio}acétique,
- acide {{[dihydro-2,3 oxo-2 phényl-5 (pyrrolidinyl-1)carbonylméthyl-1 1H-benzo[f]diazépine-1,4 yl-3-(RS)]-3 uréido}-3 phénylsulfinyl}acétique-(RS),
- acide {{[dihydro-2,3 oxo-2 phényl-5 (pyrrolidinyl-1)carbonylméthyl-1 1H-benzo[f]diazépine-1,4 yl-3]-3 uréido}-3 phénylsulfonyl}acétique-(RS),
- acide {{[dihydro-2,3 oxo-2 phényl-5 (pyrrolidinyl-1)carbonylméthyl-1 1H-benzo[f]diazépine-1,4 yl-3-(RS)]-3 uréido}-3 phénylthio}-2 propionique-(RS)
et leurs sels.

6. Procédé de préparation des composés de formule (I) selon la revendication 1 caractérisé en ce que l'on fait réagir un dérivé réactif de l'acide carbamique, obtenu éventuellement in situ par action d'un dérivé réactif de l'acide carbonique choisi parmi le N,N'-diimidazole carbonyle, le phosgène, le chloroformiate de trichlorométhyle, le carbonate de bis(trichlorométhyle) ou le chloroformiate de p-nitrophényle sur un dérivé de formule : 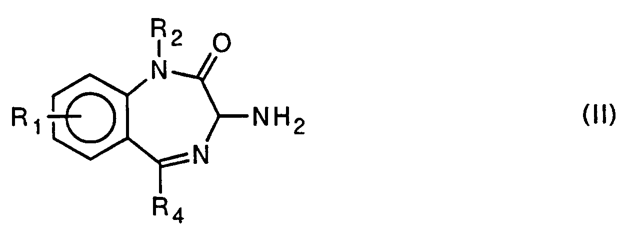 dans laquelle R₁, R₂ et R₄ ont les mêmes significations que dans la revendication 1 sur un dérivé de formule :
H₂N-R₃ (III)
dans laquelle R₃ a les mêmes significations que dans la revendication 1 ou, lorsque R₃ comporte un reste COOH, PO₃H₂, SO₂H ou SO₃H, un sel d'un tel composé puis isole le produit et le transforme éventuellement en sel.

7. Procédé de préparation des composés de formule (I) caractérisé en ce que l'on fait réagir un dérivé de formule : 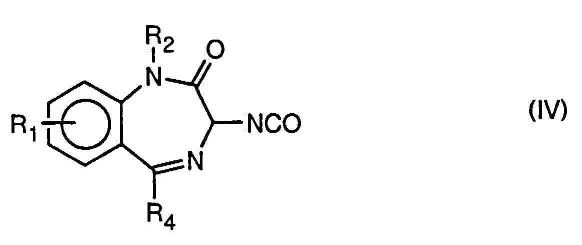 dans laquelle R₁, R₂ et R₄ ont les mêmes significations que dans la formule (I), sur une amine de formule :
H₂N-R₃ (III)
dans laquelle R₃ a les mêmes significations que dans la revendication 1 ou, lorsque R₃ comporte un reste COOH, PO₃H₂, SO₂H ou SO₃H, un sel d'un tel composé puis isole le produit et le transforme éventuellement en sel.

8. Procédé de préparation des composés de formule (I) selon la revendication 1 à l'exception de ceux pour lesquels R₃ représente soit un radical phényle substitué par -alk-PO₃H₂, -CH=NOH, -CH=N-O-alk-COOX, -S-alk-COOX, -SO-alk-COOX, -SO₂-alk-COOX, -CH=CH-COOX, -alk-CO-NHOH, -alk-N(OH)-CO-alk, -C(=NOH)-COOX, -C(COOX)=NO-alk-COOX, -alk-SO₂H ou tétrazolylalkyle soit un cycle de formule (A) dans laquelle R₉ représente un radical =NOX, =NO-alk-COOX, =CH-COOX, -alk-COOX ou -alk-SO₃H et X représente un atome d'hydrogène caractérisé en ce que l'on fait réagir un composé de formule : 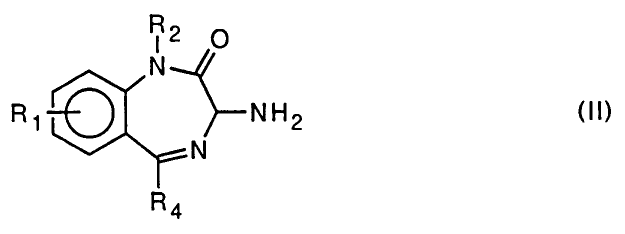 dans laquelle R₁, R₂ et R₄ ont les mêmes significations que dans la revendication 1 sur un isocyanate de formule :
OCN-R₃ (V)
dans laquelle R₃ a les mêmes significations que précédemment, isole le produit et le transforme éventuellement en sel.

9. Procédé de préparation des composés de formule (I) pour lesquels R₃ représente un radical phényle substitué par un radical -SO-alk-COOX ou SO₂-alk-COOX et X représente un radical alkyle caractérisé en ce que l'on oxyde les composés de formule (I) correspondants pour lesquels R₃ représente un radical phényle substitué par un radical -S-alk-COOX, isole le produit et le transforme éventuellement en sel.

10. Procédé de préparation des composés de formule (I) selon la revendication 1 pour lesquels R₃ représente soit un radical phényle substitué par -CH=NO-alk-COOX, -S-alk-COOX, -SO-alk-COOX, -SO₂-alk-COOX, -CH=CH-COOX, -C(COOX)=N-O-alk-COOX ou -C(=NOH)-COOX, soit un cycle de formule (A) dans laquelle R₉ représente un radical =CH-COOX, =NO-alk-COOX ou -alk-COOX et X représente un atome d'hydrogène caractérisé en ce que l'on coupe un ester correspondant, isole le produit et le transforme éventuellement en sel.

11. Procédé de préparation des composés de formule (I) pour lesquels R₃ représente un cycle (A) dans lequel R₉ représente un radical =NOX ou =NO-alk-COOX caractérisé en ce que l'on fait réagir un dérivé de formule : 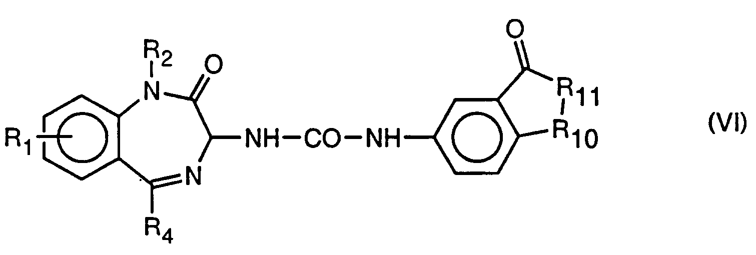 dans laquelle R₁, R₂, R₄, R₁₀ et R₁₁ ont les mêmes significations que dans la formule (I), sur un dérivé de formule :
H₂NOZ (VII)
dans laquelle Z représente un atome d'hydrogène, un radical alkyle ou -alk-COOX, isole le produit et le transforme éventuellement en sel.

12. Médicaments caractérisés en ce qu'ils contiennent comme ingrédient actif au moins un composé de formule (I) selon la revendication 1.

13. Médicaments selon la revendication 12 inhibiteurs de la CCK et de la gastrine.

## Claims

1. Compounds of formula: 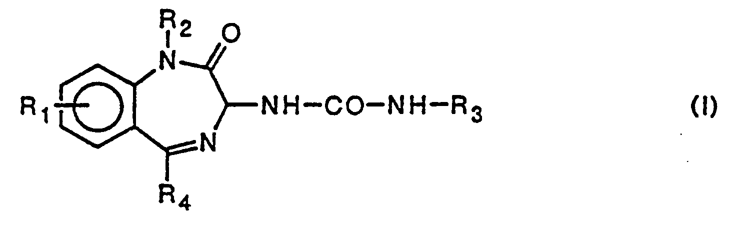 in which
- R₁ represents a hydrogen or halogen atom or an alkyl, alkoxy, alkylthio, nitro, hydroxyl or cyano radical,
- R₂ represents an alkyl radical or a chain -CH(R₅)-CO-R₆ in which R₅ represents a hydrogen atom or an alkyl or alkoxycarbonyl radical or a phenyl radical which is optionally substituted (with one or more substituents chosen from halogen atoms and alkyl, alkoxy, alkylthio and nitro radicals) and R₆ represents an alkoxy radical, a cycloalkyloxy radical which is optionally substituted (with at least one alkyl radical), a cycloalkylalkyloxy, phenylalkyloxy, polyfluoroalkyloxy or cinnamyloxy radical or a residue -NR₇R₈ in which R₇ and R₈, which may be identical or different, represent a hydrogen atom or an alkyl radical, a phenyl radical which is optionally substituted (with one or more substituents chosen from halogen atoms and alkyl, alkoxy and alkylthio radials) or a cycloalkylalkyl, cycloalkyl, indanyl or phenylalkyl radical, or alternatively R₇ and R₈, together with the nitrogen atom to which they are attached, form a saturated or unsaturated mono- or polycyclic heterocycle containing 4 to 9 carbon atoms and one or more hetero atoms (O, S, N) and optionally substituted with one or more alkyl, alkoxy, alkoxycarbonyl, dialkylcarbamoyl or phenyl radicals or with a spiromonocyclic ring system formed by combination of one atom of the heterocycle with 4 or 5 other carbon atoms, of which one or more of the latter may optionally be replaced by hetero atoms (O, S, N),
- R₃ represents (a) a phenyl radical substituted with one or more substituents chosen from the radicals -alk-SO₃H, -alk-PO₃H₂, -CH=NOH, -CH=NO-alk-COOX, -S-alk-COOX, -SO-alk-COOX, -SO₂-alk-COOX, -CH=CH-COOX, -alk-CO-NHOH, -C(=NOH)-COOX, -alk-N(OH)-CO-alk, -alk-SO₂H, -CH=CH-SO₃H, -C(COOX)=N-O-alk-COOX and tetrazolylalkyl or a group of formula: 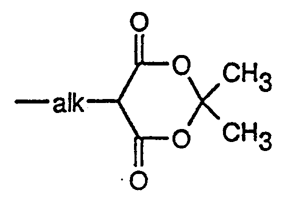 or (b) a ring system of formula: 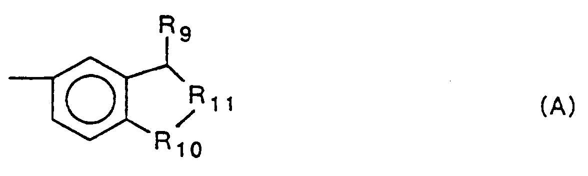 in which R₉ represents a radical =NOX, =NO-alk-COOX, =CH-COOX, -alk-COOX, -alk-SO₂H or -alk-SO₃H, R₁₀ represents an oxygen or sulphur atom or a methylene or alkylimino radical and R₁₁ represents a methylene or ethylene radical,
- R₄ represents a pyridyl or phenyl radical optionally substituted with one or more substituents chosen from halogen atoms and alkyl, alkoxy, hydroxyl, carboxyl and nitro radicals,
- alk represents an alkyl or alkylene radical,
- X represents a hydrogen atom or an alkyl radical,
with the exception of the compounds in which R₁ represents a hydrogen atom or 1 or 2 halogen or methyl, R₂ represents an alkyl radical or a radical -CH(R₅)-CO-R₆ in which R₅ represents a hydrogen atom and R₆ represents a 1-pyrrolidinyl residue, R₃ represents a phenyl radical substituted at position 3 with a 5-tetrazolylmethyl radical and R₄ represents a phenyl radical optionally substituted with one or two halogen atoms or methyl,
on the understanding that the alkyl, alkylene and alkoxy radicals and the alkyl, alkylene and alkoxy portions contain 1 to 4 carbon atoms in an unbranched or branched chain, and the cycloalkyl radicals and portions contain 3 to 12 carbon atoms,
as well as their salts and their racemates and enantiomers when they contain at least one asymmetric centre.

2. Compounds of formula (I) according to claim 1 for which R₇ and R₈, with the nitrogen atom to which they are attached, form a heterocycle chosen from piperidino, perhydro-1-azepinyl, 1,2,3,6-tetrahydro-1-pyridyl, 1,2,3,4-tetrahydro-1-quinolyl, 1-pyrrolidinyl, 1,2,3,4-tetrahydro-2-isoquinolyl, morpholino, thiomorpholino and 1-indolyl ring systems, it being possible for these ring systems to be optionally substituted with one or more alkyl radicals, their salts and their racemates and enantiomers.

3. Compounds of formula (1) according to claim 1 for which R₁ represents a hydrogen atom, R₂ represents an alkyl radical or a radical -CH(R₅)COR₆ in which R₅ represents a hydrogen atom and R₆ represents a radical -NR₇R₈ in which R₇ and R₈, together with the nitrogen atom to which they are attached, form a heterocycle, R₃ represents (a) a phenyl radical substituted with one or more substituents chosen from the radicals -alk-SO₃H, -alk-PO₃H₂, -CH=NOH, -CH=NO-alk-COOX, -S-alk-COOX, -SO-alk-COOX, -SO₂-alk-COOX, -CH=CH-COOX, -alk-CO-NHOH, -C(=NOH)-COOX, -alk-N(OH)-CO-alk, -alk-SO₂H, -CH=CH-SO₃H, -C(COOX)=N-O-alk-COOX and tetrazolylalkyl or a group of formula: 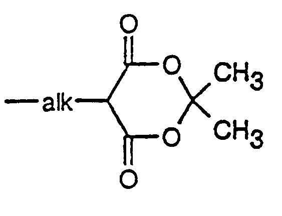 or (b) a ring system of formula: 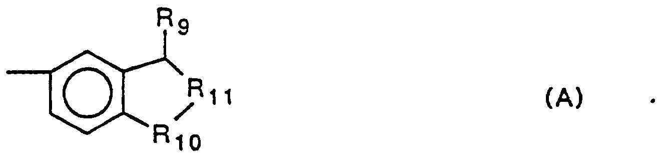 in which R₉ represents a radical =NOX, =NO-alk-COOX, =CH-COOX, -alk-COOX, -alK-SO₂H or -alk-SO₃H, R₁₀ represents an oxygen or sulphur atom or a methylene or alkylimino radical and R₁₁ represents a methylene or ethylene radical, and R₄ represents a phenyl radical.

4. Compounds of formula (I) according to claim 3 for which -NR₇R₈ represents a 1-pyrrolidinyl or piperidino ring, these rings being unsubstituted or substituted with one or more alkyl radicals, their salts and their racemates and enantiomers.

5. The following compounds:
- (RS)-{3-{3-[2,3-dihydro-2-oxo-5-phenyl-1-(1-pyrrolidinyl)carbonylmethyl-1H-1,4-benzo[f]diazepin-3-yl]ureido}phenylthio}acetic acid,
- (RS)-{3-[2,3-dihydro-1-methyl-2-oxo-5-phenyl-1H-1,4-benzo[f]diazepin-3-yl)ureido]phenylmethanesulphonic acid,
- (RS,E)-3-{3-{3-[1-(N-methyl-N-phenylcarbamoylmethyl)-2-oxo-5-phenyl-2,3-dihydro-1H-1,4-benzo[f]diazepin-3-yl]ureido}phenyl}-2-propenoic acid,
- (RS)-{3-{3-[2,3-dihydro-2-oxo-5-phenyl-1-(3,3-dimethylpiperidino)carbonylmethyl-1H-1,4-benzo[f]diazepin-3-yl]ureido}phenylthio}acetic acid,
- (RS)-{3-{3-[1-(N,N-diethylcarbamoylmethyl)-2,3-dihydro-2-oxo-5-phenyl-1H-1,4-benzo[f]diazepin-3-yl]ureido} phenylthio}acetic acid,
- (RS,E)-N-(2,3-dihydro-1-methyl-2-oxo-5-phenyl-1H-1,4-benzo[f]diazepin-3-yl)-N'-(1-hydroxyimino-6-indanyl)urea,
- (RS,E)-2-{3,4-dihydro-6-[3-(2,3-dihydro-1-methyl-2-oxo-5-phenyl-1H-1,4-benzo[f]diazepin-3-yl)ureido]-2H-4-benzopyranylidene}acetic acid,
- (RS,E)-2-{3-{3-[2,3-dihydro-2-oxo-5-phenyl-1-(1-pyrrolidinyl)carbonylmethyl-1H-1,4-benzo[f]diazepin-3-yl]-ureido}phenylmethylenaminooxy}acetic acid,
- (RS)-2-{3-{3-[2,3-dihydro-2-oxo-5-phenyl-1-(1-pyrrolidinyl)carbonylmethyl-1H-1,4-benzo[f]diazepin-3-yl]-ureido}phenyl}-2-(carboxymethyloxyimino)acetic acid,
- (RS)-5-{3-{3-[2,3-dihydro-2-oxo-5-phenyl-1-(1-pyrrolidinyl)carbonylmethyl-1H-1,4-benzo[f]diazepin-3-yl]-ureido}benzyl}-2,2-dimethyl-1,3-dioxane-4,6-dione,
- (RS,E)-2-{3-{3-[1-(N,N-diethylcarbamoylmethyl)-2,3-dihydro-2-oxo-5-phenyl-1H-1,4-benzo[f]diazepin-3-yl]ureido}phenyl}ethylenesulphonic acid,
- (RS)-5-{3-{3-[1-(N,N-diethylcarbamoylmethyl)-2,3-dihydro-2-oxo-5-phenyl-1H-1,4-benzo[f]diazepin-3-yl]ureido}benzyl}tetrazole,
- (RS,E)-3-{3-[1-(N,N-diethylcarbamoylmethyl)-2,3-dihydro-2-oxo-5-phenyl-1H-1,4-benzo[f]diazepin-3-yl]ureido}-alpha-(hydroxyimino)phenylacetic acid,
- (RS)-{3-{3-[1-(N,N-diethylcarbamoylmethyl)-2,3-dihydro-5-(2-fluorophenyl)-2-oxo-1H-1,4-benzo[f]diazepin-3-yl]ureido}phenylthio}acetic acid,
- (RS)-{4-{3-[2,3-dihydro-1-(3,3-dimethylpiperidino)carbonylmethyl-2-oxo-5-phenyl-1H-1,4-benzo[f]diazepin-3-yl]ureido}phenylthio}acetic acid,
- (RS,E)-3-{3-{3-[2,3-dihydro-2-oxo-5-phenyl-1-(3,3-dimethylpiperidino)carbonylmethyl-1H-1,4-benzo[f]diazepin-3-yl]ureido}phenyl}-2-propenoic acid,
- (RS)-{4-{3-[2,3-dihydro-2-oxo-5-phenyl-1-(1-pyrrolidonyl)carbonylmethyl-1H-1,4-benzo[f]diazepin-3-yl]ureido}phenylthio}acetic acid,
- potassium (RS)-1-{3-[3-((RS)-2,3-dihydro-2-oxo-5-phenyl-1-methyl-1H-1,4-benzo[f]diazepin-3-yl)ureido]phenyl}ethanesulphonate,
- (+)-{3-{3-[2,3-dihydro-2-oxo-5-phenyl-1-(1-pyrrolidinyl)carbonylmethyl-1H-1,4-benzo[f]diazepin-3-yl]ureido}phenylthio}acetic acid,
- (-)-{3-{3-[2,3-dihydro-2-oxo-5-phenyl-1-(1-pyrrolidinyl)carbonylmethyl-1H-1,4-benzo[f]diazepin-3-yl]ureido}phenylthio}acetic acid,
- (RS)-{3-{3-[(RS)-2,3-dihydro-2-oxo-5-phenyl-1-(1-pyrrolidinyl)carbonylmethyl-1H-1,4-benzo[f]diazepin-3-yl]ureido}phenylsulphinyl}acetic acid,
- (RS)-{3-{3-[2,3-dihydro-2-oxo-5-phenyl-1-(1-pyrrolidinyl)carbonylmethyl-1H-1,4-benzo[f]diazepin-3-yl]ureido}phenylsulphonyl}acetic acid,
- (RS)-2-{3-{3-[(RS)-2,3-dihydro-2-oxo-5-phenyl-1-(1-pyrrolidinyl)carbonylmethyl-1H-1,4-benzo[f]diazepin-3-yl]ureido}phenylthio}propionic acid,
and their salts.

6. Process for preparing the compounds of formula (I) according to claim 1, characterized in that a reactive derivative of carbamic acid, optionally obtained in situ by the action of a reactive derivative of carbonic acid chosen from N,N'-carbonyldiimidazole, phosgene, trichloromethyl chloroformate, bis(trichloromethyl) carbonate and p-nitrophenyl chloroformate on a derivative of formula: 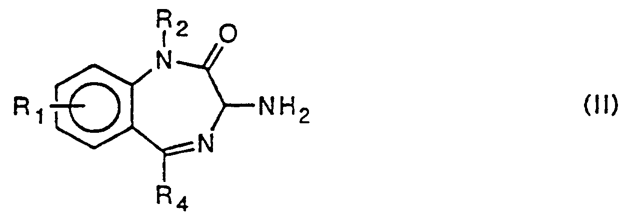 in which R₁, R₂ and R₄ have the same meanings as in claim 1, is reacted with a derivative of formula:
H₂N-R₃ (III)
in which R₃ has the same meanings as in claim 1, or, when R₃ contains a COOH, PO₃H₂, SO₂H or SO₃H residue, a salt of such a compound, and the product is then isolated and optionally converted to a salt.

7. A process for preparing the compounds of formula (I), characterized in that a derivative of formula: 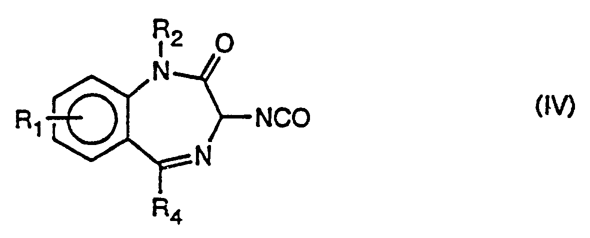 in which R₁, R₂ and R₄ have the same meanings as in the formula (I), is reacted with an amine of formula:
H₂N-R₃ (III)
in which R₃ has the same meanings as in claim 1, or, when R₃ contains a COOH, PO₃H₂, SO₂H or SO₃H residue, a salt of such a compound, and the product is then isolated and optionally converted to a salt.

8. Process for preparing the compounds of formula (I) according to claim 1, with the exception of those for which R₃ represents either a phenyl radical substituted with -alk-PO₃H₂, -CH=NOH, -CH=N-O-alk-COOX, -S-alk-COOX, -SO-alk-COOX, -SO₂-alk-COOX, -CH=CH-COOX, -alk-CO-NHOH, -alk-N(OH)-CO-alk, -C(=NOH)-COOX, -C(COOX)=NO-alk-COOX, -alk-SO₂H or tetrazolylalkyl or a ring system of formula (A) in which R₉ represents a radical =NOX, =NO-alk-COOX, =CH-COOX, -alk-COOX or -alk-SO₃H and X represents a hydrogen atom, characterized in that a compound of formula: 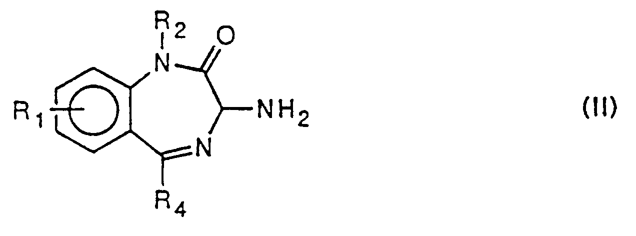 in which R₁, R₂ and R₄ have the same meanings as in claim 1, is reacted with an isocyanate of formula:
OCN-R₃ (V)
in which R₃ has the same meanings as above, and the product is isolated and optionally converted to a salt.

9. Process for preparing the compounds of formula (I) for which R₃ represents a phenyl radical substituted with a radical -SO-alk-COOX or -SO₂-alk-COOX and X represents an alkyl radical, characterized in that the corresponding compounds of formula (I) for which R₃ represents a phenyl radical substituted with a radical -S-alk-COOX are oxidized, and the product is isolated and optionally converted to a salt.

10. Process for preparing the compounds of formula (I) according to claim 1 for which R₃ represents either a phenyl radical substituted with -CH=NO-alk-COOX, -S-alk-COOX, -SO-alk-COOX, -SO₂-alk-COOX, -CH=CH-COOX, -C(COOX)=N-O-alk-COOX, or -C(=NOH)-COOX or a ring system of formula (A) in which R₉ represents a radical =CH-COOX, =NO-alk-COOX or -alk-COOX and X represents a hydrogen atom, characterized in that a corresponding ester is cleaved, and the product is isolated and optionally converted to a salt.

11. Process for preparing the compounds of formula (I) for which R₃ represents a ring system (A) in which R₉ represents a radical =NOX or =NO-alk-COOX, characterized in that a derivative of formula: 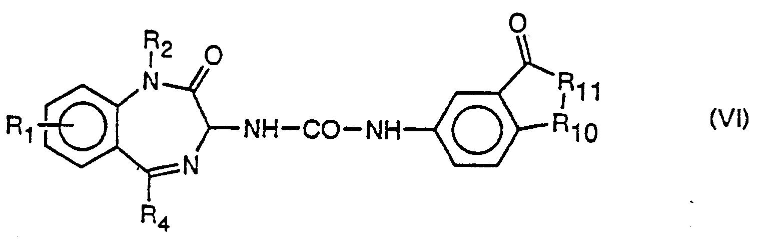 in which R₁, R₂, R₄, R₁₀ and R₁₁ have the same meanings as in the formula (I), is reacted with a derivative of formula:
H₂NOZ (VII)
in which Z represents a hydrogen atom, an alkyl radical or a radical -alk-COOX, and the product is isolated and optionally converted to a salt.

12. Medicinal products, characterized in that they contain as active ingredient at least one compound of formula (I) according to claim 1.

13. Medicinal products according to claim 12, which are CCK and gastrin inhibitors.

## Patentansprüche

1. Verbindungen der Formel 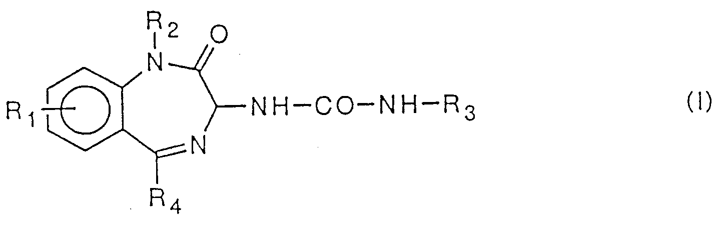 worin
- R₁ einen Wasserstoff- oder Halogenatom oder einen Alkyl-, Alkoxy-, Alkylthio-, Nitro-, Hydroxy- oder Cyanorest bedeutet,
- R₂ einen Alkylrest oder eine Kette -CH(R₅)-CO-R₆ darstellt, worin R₅ ein Wasserstoffatom oder einen Alkyl-, Alkoxycarbonyl- oder Phenylrest bedeutet, der gegebenenfalls substituiert ist (durch einen oder mehrere Substituenten ausgewählt unter den Halogenatomen und den Alkyl-, Alkoxy-, Alkylthio- und Nitroresten), und R₆ bedeutet einen Alkoxy-, Cycloalkyloxyrest, der gegebenenfalls substituiert ist (durch mindestens einen Alkylrest), Cycloalkylalkyloxy, Phenylalkyloxy, Polyfluoroalkyloxy, Cinnamyloxy, einen Rest -NR₇R₈, worin R₇ und R₈, die identisch oder verschieden sind, ein Wasserstoffatom oder einen Alkylrest, Phenyl gegebenenfalls substituiert (durch einen oder mehrere Substituenten ausgewählt unter den Halogenatomen und den Alkyl-, Alkoxy- und Alkylthioresten), Cycloalkylalkyl, Cycloalkyl, Indanyl, Phenylalkyl oder auch bilden R₇ und R₈ zusammen mit dem Stickstoffatom, an das sie gebunden sind, einen gesättigten oder ungesättigten mono- oder polycyclischen Heterozyklus, enthaltend 4 bis 9 Kohlenstoffatome und ein oder mehrere Heteroatome (O, S, N) und gegebenenfalls substituiert durch einen oder mehrere Alkyl-, Alkoxy-, Alkoxycarbonyl-, Dialkylcarbamoyl-, Phenylrest(e) oder einen spiromonocyclischen Ring, gebildet durch Kombination eines Atoms des Heterozyklus mit 4 oder 5 anderen Kohlenstoffatomen, wovon eines oder mehrere dieser letzteren gegebenenfalls durch Heteroatome (O, S, N) ersetzt sein können,
- R₃ bedeutet (a) einen Phenylrest substituiert durch einen oder mehrere Substituenten ausgewählt unter den Resten -alk-SO₃H -alk-PO₃H₂, -CH=NOH, -CH=NO-alk-COOX, -C(=NOH)-COOX, -S-alk-COOX, -SO-alk-COOX, -SO₂-alk-COOX, -CH=CH-COOX, -alk-CO-NHOH, -alk-N(OH)-CO-alk, -alk-SO₂H, -CH=CH-SO₃H, -C(COOX)=N-O-alk-COOX, Tetrazolylalkyl oder eine Gruppe der Formel 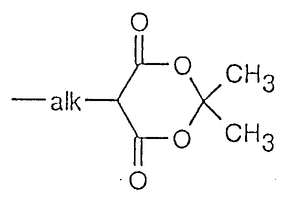 oder (b) einen Ring der Formel 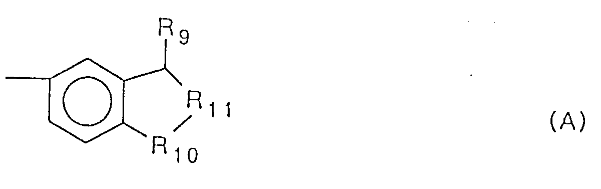 worin R₉ einen Rest =NOX, =NO-alk-COOX, =CH-COOX, -alk-COOX, -alk-SO₂H oder -alk-SO₃H bedeutet, R₁₀ bedeutet ein Sauerstoff- oder Schwefelatom oder einen Methylen- oder Alkyliminorest, und R₁₁ bedeutet einen Methylen- oder Ethylenrest,
- R₄ bedeutet einen Pyridylrest oder Phenyl, gegebenenfalls substituiert durch einen oder mehrere Substituenten, ausgewählt unter den Halogenatomen und den Alkyl-, Alkoxy-, Hydroxy-, Carboxy- und Nitroresten,
- alk bedeutet einen Alkyl- oder Alkylenrest,
- X bedeutet ein Wasserstoffatom oder einen Alkylrest,
mit Ausnahme der Verbindungen, für die R₁ ein Wasserstoffatom oder 1 oder 2 Halogenatome oder Methyl bedeutet, R₂ einen Alkylrest oder -CH(R₅)-CO-R₆ bedeutet, worin R₅ ein Wasserstoffatom und R₆ einen Pyrrolidin-1-ylrest darstellt, R₃ einen Phenylrest substituiert in 3-Stellung durch einen Rest Tetrazolyl-5-methyl und R₄ einen Phenylrest gegebenenfalls substituiert durch ein oder zwei Halogenatome oder Methyl darstellt,
wobei die Alkyl-, Alkylen- und Alkoxyreste und die Alkyl-, Alkylen- und Alkoxyteile 1 bis 4 Kohlenstoffatome in gerader oder verzweigter Kette enthalten und die Cycloalkylteile und -reste 3 bis 12 Kohlenstoffatome enthalten,
sowie ihre Salze, ihre Racemate und Enantiomeren, wenn sie mindestens ein asymmetrisches Zentrum enthalten.

2. Verbindungen der Formel (I) gemäß Anspruch 1, für die R₇ und R₈ mit dem Stickstoffatom, an das sie gebunden sind, einen Heterozyklus bilden, ausgewählt unter den Ringen Piperidino, Perhydroazepin-1-yl, 1,2,3,6-Tetrahydro-1-pyridyl, 1,2,3,4-Tetrahydro-chinolyl-1, Pyrrolidin-1-yl, 1,2,3,4-Tetrahydro-isochinolyl-2, Morpholino, Thiomorpholino oder Indolyl-1, wobei diese Ringe gegebenenfalls durch einen oder mehrere Alkylreste substituiert sein können, ihre Salze, ihre Racemate und Enantiomeren.

3. Verbindungen der Formel (I) gemäß Anspruch 1, für die R₁ ein Wasserstoffatom bedeutet, R₂ einen Alkylrest oder -CH(R₅)COR₆ darstellt, worin R₅ ein Wasserstoffatom bedeutet und R₆ einen Rest -NR₇R₈ bedeutet, worin R₇ und R₈ zusammen mit dem Stickstoffatom, an das sie gebunden sind, einen Heterozyklus bilden, R₃ bedeutet (a) einen Phenylrest substituiert durch einen oder mehrere Substituenten ausgewählt unter den Resten -alk-SO₃H, -alk-PO₃H₂, -CH=NOH, -CH=NO-alk-COOX, -S-alk-COOX, -SO-alk-COOX, -SO₂-alk-COOX, -CH=CH-COOX, -alk-CO-NHOH, -C(=NOH)-COOX, -alk-N(OH)-CO-alk, -alk-SO₂H,-CH=CH-SO₃H, -C(COOX)=N-O-alk-COOX, Tetrazolylalkyl oder eine Gruppe der Formel 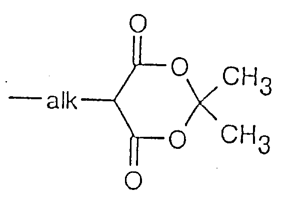 oder (b) einen Ring der Formel 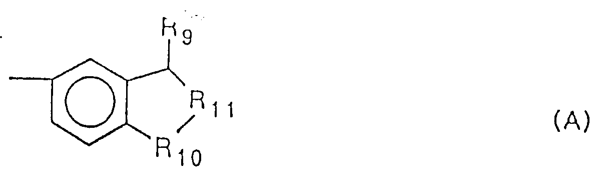 worin R₉ einen Rest =NOX, =NO-alk-COOX, =CH-COOX, -alk-COOX, -alk-SO₂H oder -alk-SO₃H bedeutet, R₁₀ bedeutet ein Sauerstoff- oder Schwefelatom oder einen Methylen- oder Alkyliminorest, und R₁₁ bedeutet einen Methylen- oder Ethylenrest und R₄ bedeutet einen Phenylrest.

4. Verbindungen der Formel (I) gemäß Anspruch 3, für die -NR₇R₈ einen Pyrrolidin-1-ylring oder Piperidino bedeutet, wobei diese Ringe nicht substituiert sind oder substituiert sind durch einen oder mehrere Alkylreste,ihre Salze, ihre Racemate und Enantiomeren.

5. Die folgenden Verbindungen:
(RS)-{3-{3-[2,3-Dihydro-2-oxo-5-phenyl-1-(pyrrolidin-1-yl)-carbonylmethyl-1H-1,4-benzo[f]diazepin-3-yl]-ureido}-phenylthio}-essigsäure,
(RS)-3-[3-(2,3-Dihydro-1-methyl-2-oxo-5-phenyl-1H-1,4-benzo [f]diazepin-3-yl)-ureido]-phenylmethansulfonsäure,
(RS)-3-{3-[1-(N-Methyl-N-phenyl-carbamoylmethyl)-2-oxo-5-phenyl-2,3-dihydro-1H-1,4-benzo[f]diazepin-3-yl]-ureido}-3-phenyl-2(E)-propensäure
(RS)-{3-{3-[2,3-Dihydro-2-oxo-5-phenyl-1-(3,3-dimethylpiperidino)-carbonylmethyl-1H-1,4-benzo[f]diazepin-3-yl]-ureido}-phenylthio}-essigsäure,
(RS)-{3-{3-[1-(N,N-Diethyl-carbamoylmethyl)-2,3-dihydro-2-oxo-5-phenyl-1H-1,4-benzo[f]diazepin-3-yl]-ureido}-phenylthio}-essigsäure,
(RS)-(E)-N-(2,3-Dihydro-1-methyl-2-oxo-5-phenyl-1H-1,4-benzo[f]diazepin-3-yl)-N'-(1-hydroxyimino-6-indanyl)-harnstoff,
(RS)-(E)-2-{3,4-Dihydro-6-[3-(2,3-dihydro-1-methyl-2-oxo-5-phenyl-1H-1,4-benzo[f]diazepin-3-yl)-ureido]-2H-benzopyran nyliden-4}-essigsäure,
(RS)-(E)-3-{3-[2,3-Dihydro-2-oxo-5-phenyl-1-(pyrrolidin-1-yl)-carbonylmethyl-1H-1,4-benzo[f]diazepin-3-yl]-ureido}-2-phenylmethylenaminooxy-essigsäure,
(RS)-2-{3-{3-[(2,3-Dihydro-2-oxo-5-phenyl-1-(pyrrolidin-1-yl)-carbonylmethyl-1H-1,4-benzo[f]diazepin-3-yl]-ureido}-phenyl}-2-(carboxymethyloxyimino)-essigsäure,
(RS)-5-{3-{3-[2,3-Dihydro-2-oxo-5-phenyl-1-(pyrrolidin-1-yl)-carbonylmethyl-1H-1,4-benzo[f]diazepin-3-yl]-ureido}-benzyl}-2,2-dimethyl-1,3-dioxan-4,6-dion,
(RS)-(E)-2-{3-{3-[1-(N,N-Diethylcarbamoylmethyl)-2,3-dihydro-2-oxo-5-phenyl-1H-1,4-benzo[f]diazepin-3-yl]-ureido}-phenyl}-ethylensulfonsäure,
(RS)-5-{3-{3-[1-(N,N-Diethyl-carbamoylmethyl)-2,3-dihydro-2-oxo-5-phenyl-1H-1,4-benzo[f]diazepin-3-yl]-ureido}-benzyl}-tetrazol,
(RS)-(E)-3-{3-[1-(N,N-Diethyl-carbamoylmethyl)-2,3-dihydro-2-oxo-5-phenyl-1H-1,4-benzo[f]diazepin-3-yl]-ureido}-alpha-hydroxyimino-phenylessigsäure,
(RS)-{3-{3-[1-(N,N-Diethyl-carbamoylmethyl)-2,3-dihydro-5-(2-fluorophenyl)-2-oxo-1H-1,4-benzo[f]diazepin-3-yl]-ureido}-phenylthio}-essigsäure,
(RS)-{4-{3-[2,3-Dihydro-1-(3,3-dimethylpiperidino)-carbonylmethyl-2-oxo-5-phenyl-1H-1,4-benzo[f]diazepin-3-yl]-ureido}-phenylthio}-essigsäure,
(RS)-(E)-3-{3-{3-[2,3-Dihydro-2-oxo-5-phenyl-1-(3,3-dimethylpiperidino)-carbonylmethyl-1H-1,4-benzo[f]diazepin-3-yl]-ureido}-phenyl}-2-propensäure,
(RS)-{4-{3-[2,3-Dihydro-2-oxo-5-phenyl-1-(pyrrolidin-1-yl)-carbonylmethyl-1H-1,4-benzo[f]diazepin-3-yl]-ureido}-phenylthio}-essigsäure,
(RS)-Kalium-1-{3-[3-((RS)-2,3-dihydro-2-oxo-5-phenyl-1-methyl-1H-1,4-benzo[f]diazepin-3-yl)-ureido]-phenyl}-ethansulfonat,
(+)-{3-{3-[2,3-Dihydro-2-oxo-5-phenyl-1-(pyrrolidin-1-yl)-carbonylmethyl-1H-1,4-benzo[f]diazepin-3-yl]-ureido}-phenylthio}-essigsäure,
(-)-{3-{3-[2,3-Dihydro-2-oxo-5-phenyl-1-(pyrrolidin-1-yl)-carbonylmethyl-1H-1,4-benzo[f]diazepin-3-yl]-ureido}-phenylthio}-essigsäure,
(RS)-{3-{3-[(RS)-2,3-Dihydro-2-oxo-5-phenyl-1-(pyrrolidin-1-yl)-carbonylmethyl-1H-1,4-benzo[f]diazepin-3-yl]-ureido}-phenylsulfinyl}-essigsäure,
(RS)-{3-{3-[2,3-Dihydro-2-oxo-5-phenyl-1-(pyrrolidin-1-yl)-carbonylmethyl-1H-1,4-benzo[f]diazepin-3-yl]-ureido}-phenylsulfonyl}-essigsäure,
(RS)-2-{3-{3-[(RS)-2,3-Dihydro-2-oxo-5-phenyl-1-(pyrrolidin-1-yl)-carbonylmethyl-1H-1,4-benzo[f]diazepin-3-yl]-ureido}-phenylthio}-propionsäure,
und ihre Salze.

6. Verfahren zur Herstellung der Verbindungen der Formel (I) gemäß Anspruch 1, dadurch gekennzeichnet, daß man zur Reaktion bringt ein reaktionsfähiges Derivat der Carbamidsäure, erhalten gegebenenfalls in situ durch Einwirkung eines reaktionsfähigen Derivats der Kohlensäure ausgewählt unter N,N'-Diimidazolcarbonyl, Phosgen, Trichloromethylchlorformiat, Bis(trichlormethyl)-carbonat oder p-Nitrophenylchlorformiat auf ein Derivat der Formel 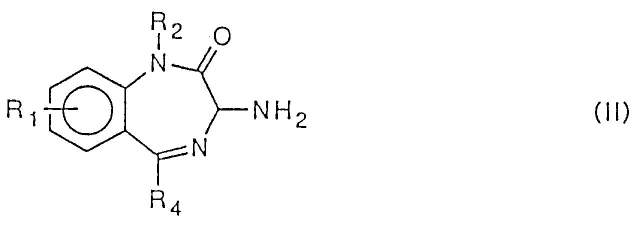 worin R₁, R₂ und R₄ die gleichen Bedeutungen wie in Anspruch 1 haben, mit einem Derivat der Formel
H₂N-R₃ (III)
worin R₃ die gleichen Bedeutungen wie in Anspruch 1 hat, oder wenn R₃ einen Rest COOH, PO₃H₂, SO₂H oder SO₃H enthält, ein Salz einer solchen Verbindung, dann das Produkt isoliert und es gegebenenfalls in ein Salz überführt.

7. Verfahren zur Herstellung der Verbindungen der Formel (I), dadurch gekennzeichnet, daß man ein Derivat der Formel 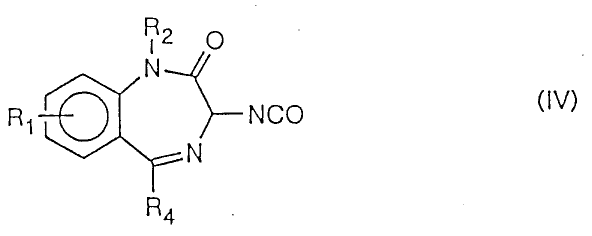 worin R₁, R₂ und R₄ die gleichen Bedeutungen wie in Formel (I) hat, mit einem Amin der Formel
H₂N-R₃ (III)
zur Reaktion bringt, worin R₃ die gleichen Bedeutungen wie in Anspruch 1 hat, oder wenn R₃ einen Rest COOH, PO₃H₂, SO₂H oder SO₃H aufweist, ein Salz einer solchen Verbindung, dann das Produkt isoliert und es gegebenenfalls in ein Salz überführt.

8. Verfahren zur Herstellung der Verbindungen der Formel (I) gemäß Anspruch 1, mit Ausnahme solcher, für die R₃ entweder einen Phenylrest substituiert durch -alk-PO₃H₂, -CH=NOH, -CH=N-O-alk-COOX, -S-alk-COOX, -SO-alk-COOX, -SO₂-alk-COOX, -CH=CH-COOX, -alk-CO-NHOH, -alk-N(OH)-CO-alk, -C(=NOH)-COOX, -C(COOX)=NO-alk-COOX, -alk-SO₂H oder Tetrazolylalkyl oder einen Ring der Formel (A) bedeutet, worin R₉ einen Rest =NOX, =NO-alk-COOX, =CH-COOX, -alk-COOX oder -alk-SO₃H bedeutet, und X ein Wasserstoffatom bedeutet, dadurch gekennzeichnet, daß man eine Verbindung der Formel 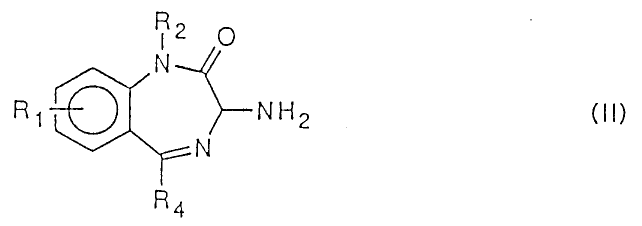 worin R₁, R₂ und R₄ die gleichen Bedeutungen wie in Anspruch haben, mit einem Isocyanat der Formel
OCN-R₃ (V)
worin R₃ die gleichen Bedeutung wie vorstehend hat, zur Reaktion bringt, das Produkt isoliert und es gegebenenfalls in ein Salz überführt.

9. Verfahren zur Herstellung der Verbindungen der Formel (I), für die R₃ einen Phenylrest substituiert durch einen Rest -SO-alk-COOX oder -SO₂-alk-COOX bedeutet, und X einen Alkylrest darstellt, dadurch gekennzeichnet, daß man die entsprechenden Verbindungen der Formel (I), für die R₃ einen Phenylrest substituiert durch einen Rest -S-alk-COOX bedeutet, oxidiert, das Produkt isoliert und es gegebenenfalls in ein Salz überführt.

10. Verfahren zur Herstellung der Verbindungen der Formel (I) gemäß Anspruch 1, für die R₃ entweder einen Phenylrest substituiert durch -CH=NO-alk-COOX, -S-alk-COOX, -SO-alk-COOX, -SO₂-alk-COOX, -CH=CH-COOX, -C(COOX)=N-O-alk-COOX oder -C(=NOH)-COOX oder einen Ring der Formel (A) bedeutet, worin R₉ einen Rest =CH-COOX, =NO-alk-COOX oder -alk-COOX bedeutet, und X ein Wasserstoffatom darstellt, dadurch gekennzeichnet, daß man einen entsprechenden Ester zerlegt, das Produkt isoliert und es gegebenenfalls in ein Salz überführt.

11. Verfahren zur Herstellung der Verbindungen der Formel (I), für die R₃ einen Ring (A) bedeutet, worin R₉ einen Rest =NOX oder =NO-alk-COOX darstellt, dadurch gekennzeichnet, ddaß man ein Derivat der Formel 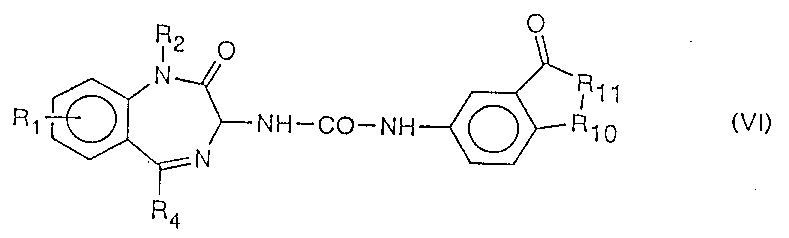 worin R₁, R₂, R₄, R₁₀ und R₁₁ die gleichen Bedeutungen wie in Formel (I) haben, mit einem Derivat der Formel
H₂NOZ (VII)
worin Z ein Wasserstoffatom,einen Alkylrest oder -alk-COOX bedeutet, zur Reaktion bringt, das Produkt isoliert und es gegebenenfalls in ein Salz überführt.

12. Arzneimittel, dadurch gekennzeichnet, daß sie als Wirkstoff mindestens eine Verbindung der Formel (I) gemäß Anspruch 1 enthalten.

13. Arzneimittel gemäß Anspruch 12, Inhibitoren von CCK und von Gastrin.
